# EUROPÄISCHE PATENTSCHRIFT

(11) **EP 1 592 669 B1**
(45) Veröffentlichungstag und Bekanntmachung des Hinweises auf die Patenterteilung: **17.10.2007**
(21) Anmeldenummer: 04703371.7
(22) Anmeldetag: 20.01.2004
(51) Int. Cl.: C07D 233/50, C07D 235/30, A61K 31/4168, A61K 31/4184, A61P 1/10, A61P 9/12, A61P 11/16, A61P 13/12, A61P 33/02, A61P 39/00

(54) **SUBSTITUIERTE 2-AMINOIMIDAZOLE, VERFAHREN ZU IHRER HERSTELLUNG, IHRE VERWENDUNG ALS MEDIKAMENT ODER DIAGNOSTIKUM SOWIE DIESE ENTHALTENDES MEDIKAMENT**
SUBSTITUTED 2-AMINOIMIDAZOLES, METHOD FOR THE PRODUCTION THEREOF, THEIR USE AS A MEDICAMENT OR AS A DIAGNOSTIC REAGENT, AND MEDICAMENT CONTAINING THESE COMPOUNDS
2-AMINO-IMIDAZOLES SUBSTITUES, PROCEDES DE PREPARATION CORRESPONDANTS ET UTILISATION COMME MEDICAMENT OU COMME AGENT DIAGNOSTIQUE ET MEDICAMENT LES CONTENANT

(30) Priorität: 04.02.2003 DE 10304374
(43) Veröffentlichungstag der Anmeldung: 09.11.2005
(60) Teilanmeldung: 07014956.2
(73) Patentinhaber: Sanofi-Aventis Deutschland GmbH, 65929 Frankfurt am Main (DE)
(72) Erfinder: HEINELT, Uwe, 65187 Wiesbaden (DE); LANG, Hans-Jochen, 65719 Hofheim (DE); HOFMEISTER, Armin, 65926 Frankfurt (DE); WIRTH, Klaus, 65926 Frankfurt (DE); JANSEN, Hans-Willi, 65527 Niedernhausen (DE)
(86) Internationale Anmeldenummer: PCT/EP2004/000394
(87) Internationale Veröffentlichungsnummer: WO 2004/069806

(56) Entgegenhaltungen:
- WO-A-02/46169
- US-A- 4 275 072

## Beschreibung

Die Erfindung betrifft Verbindungen vom Typ der 2-Aminoimidazole, die den Natrium-Protonen-Austauscher, insbesondere den Subtyp 3 (NHE3), hemmen und die nützlich bei der Prävention oder Behandlung diverser Erkrankungen sind. So lassen sich die Verbindungen unter anderem bei Nierenerkrankungen wie akutem oder chronischem Nierenversagen, bei Störungen der Gallenfunktion, bei Atemstörungen wie Schnarchen oder Schlafapnoen oder bei Schlaganfall einsetzen.

Die Erfindung betrifft Verbindungen der Formel I, 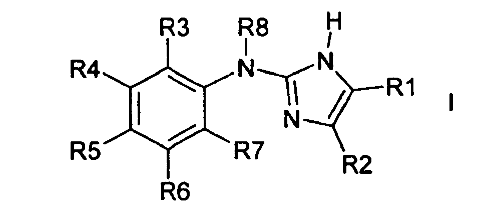 worin bedeuten:
- R1 und R2: zusammen mit den beiden Kohlenstoffatomen, an die sie gebunden sind, einen fünf-, sechs-, sieben- oder achtgliedrigen, eine oder zwei Doppelbindungen enthaltenden Kohlenstoffring,
wobei der Ring unsubstituiert ist oder mit 1, 2, 3, 4, 5, 6, 7, 8, 9, 10, 11 oder 12 F-Atomen und/oder mit einem oder zwei Resten aus der Gruppe OH, NR9R10, Alkyl mit 1, 2, 3 oder 4 C-Atomen, CN, CF₃ oder Alkoxy mit 1, 2, 3 oder 4 C-Atomen substituiert ist;
R9 und R10 unabhängig voneinander H oder Alkyl mit 1, 2, 3 oder 4 C-Atomen;
- R3, R4, R5, R6 und R7: unabhängig voneinander H, F, Cl, Br, I, Alkyl mit 1, 2, 3 oder 4 C-Atomen, oder Alkoxy mit 1, 2, 3 oder 4 C-Atomen,
wobei die Kohlenstoffketten unsubstituiert oder unabhängig voneinander mit 1, 2, 3, 4, 5, 6, 7, 8 oder 9 F-Atomen substituiert sind,
- R8: wobei R3 und R7 nicht beide gleichzeitig Wasserstoff entsprechen; H, Alkyl mit 1, 2, 3 oder 4 C-Atomen oder Cycloalkyl mit 3, 4 oder 5 C-Atomen, wobei die Kohlenstoffketten oder Cycloalkylreste unsubstituiert oder mit 1, 2, 3, 4, 5, 6, 7, 8 oder 9 F-Atomen substituiert sind;
sowie deren pharmazeutisch verträgliche Salze und Trifluoressigsäuresalze.

Bevorzugt sind Verbindungen der Formel I, worin bedeuten
- R1 und R2: zusammen mit den beiden Kohlenstoffatomen, an die sie gebunden sind, einen fünf-, sechs-, sieben- oder achtgliedrigen, eine oder zwei Doppelbindungen enthaltenden Kohlenstoffring,
wobei der Ring unsubstituiert oder mit 1, 2, 3, 4, 5, 6, 7, 8, 9, 10, 11 oder 12 F-Atomen und/oder mit ein oder zwei Resten aus der Gruppe CH₃ oder OCH₃ substituiert ist;
- R3, R4, R5, R6 und R7: unabhängig voneinander H, F, Cl, Br, Alkyl mit 1, 2, 3 oder 4 C-Atomen oder Alkoxy mit 1, 2, 3 oder 4 C-Atomen,
wobei die Kohlenstoffketten unsubstituiert oder unabhängig voneinander mit 1, 2, 3, 4, 5, 6, 7, 8 oder 9 F-Atomen substituiert sind,
wobei R3 und R7 nicht beide gleichzeitig Wasserstoff entsprechen;
- R8: H, Alkyl mit 1, 2, 3 oder 4 C-Atomen oder Cycloalkyl mit 3, 4 oder 5 C-Atomen,
wobei die Kohlenstoffketten oder Cycloalkylreste unsubstituiert oder mit 1, 2, 3, 4, 5, 6, 7, 8 oder 9 F-Atomen substituiert sind;
sowie deren pharmazeutisch verträgliche Salze und Trifluoressigsäuresalze.

Besonders bevorzugt sind Verbindungen der Formel I, worin bedeuten:
- R1 und R2: zusammen mit den beiden Kohlenstoffatomen, an die sie gebunden sind, einen fünf-, sechs- oder siebengliedrigen, eine Doppelbindung enthaltenden Kohlenstoffring,
- R3 und R7: unabhängig voneinander F, Cl, Br oder Alkyl mit 1, 2, 3 oder 4 C-Atomen, wobei die Kohlenstoffketten unsubstituiert oder unabhängig voneinander mit 1, 2, 3, 4, 5, 6, 7, 8 oder 9 F-Atomen substituiert sind;
- R4, R5 und R6: H
- R8: H oder Alkyl mit 1, 2, 3 oder 4 C-Atomen,
wobei die Kohlenstoffketten unsubstituiert oder mit 1, 2, 3, 4, 5, 6, 7, 8 oder 9 F-Atomen substituiert sind;
sowie deren pharmazeutisch verträgliche Salze und Trifluoressigsäuresalze.

Ganz besonders bevorzugt sind:
(2,6-Dichlorphenyl)-(4,5,6,7-tetrahydro-1H-benzoimidazol-2-yl)-amin- oder
(2,6-Dichlorphenyl)-methyl-(4,5,6,7-tetrahydro-1H-benzoimidazol-2-yl)-amin
sowie deren pharmazeutisch verträgliche Salze und Trifluoressigsäuresalze.

In einer Ausführungsform sind Verbindungen der Formel I bevorzugt, in denen R1 und R2 zusammen mit den beiden Kohlenstoffatomen, an die sie gebunden sind, einen fünf-, sechs-, sieben- oder achtgliedrigen, eine oder zwei Doppelbindungen enthaltenden Kohlenstoffring bilden, wobei der Ring unsubstituiert oder durch1, 2, 3, 4, 5, 6, 7, 8, 9, 10, oder 11 F-Atome und/oder mit einem oder zwei Resten aus der Gruppe CH₃ oder OCH₃ substituiert ist; besonders bevorzugt sind Verbindungen, in denen R1 und R2 zusammen mit den beiden Kohlenstoffatomen, an die sie gebunden sind, einen fünf-, sechs- oder siebengliedrigen, eine Doppelbindung enthaltenden, unsubstituierten Kohlenstoffring bilden.
In einer weiteren Ausführungsform sind Verbindungen der Formel l bevorzugt, in denen R3 und R7 nicht durch Wasserstoff beschrieben werden; besonders bevorzugt sind Verbindungen, in denen R3 und R7 unabhängig voneinander durch F, Cl, Br oder Alkyl mit 1, 2, 3 oder 4 C-Atomen beschrieben werden, wobei die Kohlenstoffketten unsubstituiert oder unabhängig voneinander mit 1, 2, 3, 4, 5, 6, 7, 8 oder 9 F-Atomen substituiert sind; ganz besonders bevorzugt sind Verbindungen, in denen R3 und R7 unabhängig voneinander durch Cl oder Methyl, insbesondere Cl beschrieben werden. In einer weiteren Ausführungsform sind Verbindungen der Formel I bevorzugt, in denen R4, R5 und R6 durch Wasserstoff beschrieben werden.
In einer weiteren Ausführungsform sind Verbindungen der Formel I bevorzugt, in denen R8 durch Wasserstoff oder Alkyl mit 1, 2, 3 oder 4 C-Atomen beschrieben wird, besonders bevorzugt sind Verbindungen, in denen R8 durch Wasserstoff oder Methyl beschrieben wird.

Weiterhin umfasst die Erfindung die Verwendung von Verbindungen der Formel I 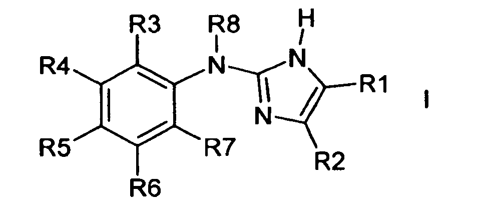 zur Herstellung eines Medikaments zur Behandlung von Krankheiten, welche durch Inhibierung des Natrium/Wasserstoff-Austauschers (NHE), insbesondere des NHE3, beeinflusst werden können, worin bedeuten:
- R1 und R2: zusammen mit den beiden Kohlenstoffatomen, an die sie gebunden sind, einen fünf-, sechs-, sieben- oder achtgliedrigen, eine oder zwei Doppelbindungen enthaltenden Kohlenstoffring,
wobei der Ring unsubstituiert ist oder mit 1, 2, 3, 4, 5, 6, 7, 8, 9, 10, 11 oder 12 F-Atomen und/oder ein oder zwei Resten aus der Gruppe NR9R10, Alkyl mit 1, 2, 3 oder 4 C-Atomen, OH, CN, CF₃ oder Alkoxy mit 1, 2, 3 oder 4 C-Atomen substituiert ist mit
R9, R10 unabhängig voneinander H, Alkyl mit 1, 2, 3 oder 4 C-Atomen;
- R3, R4, R5, R6 oder R7: unabhängig voneinander H, F, Cl, Br, l, Alkyl mit 1, 2, 3 oder 4 C-Atomen, (C₂-C₄)-Alkenyl, Cycloalkyl mit 3, 4, 5 oder 6 C-Atomen, OH, Alkoxy mit 1, 2, 3 oder 4 C-Atomen, CN, NO₂, NH₂, Alkylamino mit 1, 2, 3 oder 4 C-Atomen oder Dialkylamino mit jeweils 1, 2, 3 oder 4 C-Atomen,
wobei die Kohlenstoffketten oder -ringe unsubstituiert oder unabhängig
voneinander mit 1, 2, 3, 4, 5, 6, 7, 8 oder 9 F-Atomen substituiert sind;
wobei R3 und R7 nicht gleichzeitig Wasserstoff entsprechen;
- R8: H, Alkyl mit 1, 2, 3 oder 4 C-Atomen oder Cycloalkyl mit 3, 4 oder 5 C-Atomen, wobei die Kohlenstoffketten oder -ringe unsubstituiert oder mit 1, 2, 3, 4, 5, 6, 7, 8 oder 9 F-Atomen substituiert sind;
sowie deren pharmazeutisch verträgliche Salze.

In einer Ausführungsform ist die Verwendung von Verbindungen der Formel I bevorzugt, in denen R1 und R2 zusammen mit den beiden Kohlenstoffatomen, an die sie gebunden sind, einen fünf-, sechs-, sieben- oder achtgliedrigen, eine oder zwei Doppelbindungen enthaltenden Kohlenstoffring bilden, wobei der Ring unsubstituiert oder durch 1, 2, 3, 4, 5, 6, 7, 8, 9, 10, oder 11 F-Atome und/oder mit einem oder zwei Resten aus der Gruppe CH₃ oder OCH₃ substituiert ist; besonders bevorzugt ist die Verwendung von Verbindungen, in denen R1 und R2 zusammen mit den beiden Kohlenstoffatomen, an die sie gebunden sind, einen fünf-, sechs- oder siebengliedrigen, eine Doppelbindung enthaltenden, unsubstituierten Kohlenstoffring bilden.
In einer weiteren Ausführungsform ist die Verwendung von Verbindungen der Formel I bevorzugt, in denen R3 und R7 nicht durch Wasserstoff beschrieben werden; besonders bevorzugt ist die Verwendung von Verbindungen, in denen R3 und R7 unabhängig voneinander durch F, Cl, Br oder Alkyl mit 1, 2, 3 oder 4 C-Atomen beschrieben werden, wobei die Kohlenstoffketten unsubstituiert oder unabhängig voneinander mit 1, 2, 3, 4, 5, 6, 7, 8 oder 9 F-Atomen substituiert sind; ganz besonders bevorzugt ist die Verwendung von Verbindungen, in denen R3 und R7 unabhängig voneinander durch Cl oder Methyl, insbesondere Cl beschrieben werden.
In einer weiteren Ausführungsform ist die Verwendung von Verbindungen der Formel I bevorzugt, in denen R4, R5 und R6 durch Wasserstoff beschrieben werden.

In einer weiteren Ausführungsform ist die Verwendung von Verbindungen der Formel I bevorzugt, in denen R8 durch Wasserstoff oder Alkyl mit 1, 2, 3 oder 4 C-Atomen beschrieben wird; besonders bevorzugt ist die Verwendung von Verbindungen, in denen R8 durch Wasserstoff oder Methyl beschrieben wird.

Enthalten die Substituenten R1, R2, R3, R4, R5, R6, R7 oder R8 ein oder mehrere Asymmetriezentren, so können diese unabhängig voneinander sowohl S als auch R konfiguriert sein. Die Verbindungen können als optische Isomere, als Diastereomere, als Racemate oder als Gemische derselben in allen Verhältnissen vorliegen.

Die vorliegende Erfindung umfasst alle tautomeren Formen der Verbindungen der Formel I.

Kohlenstoffketten sind alle Reste, die Kohlenstoffatome in geradkettiger oder verzweigter Anordnung enthalten, beispielweise 1, 2, 3, 4, 5 oder 6 Kohlenstoffatome. Beispiele sind Alkylreste, Alkoxyreste, Alkenylreste, Alkinylreste, Alkylaminoreste oder Dialkylaminoreste. Kohlenstoffringe sind alle Reste, die Kohlenstoffatome enthalten, die einen Ring bilden, beispielweise aus 3, 4, 5 oder 6 Kohlenstoffatomen. Beispiele für Kohlenstoffringe sind Cycloalkylreste, oder Cycloalkenylreste. Kohlenstoffketten oder -ringe können in unterschiedlichen Positionen ungesättigt und auch mehrfach ungesättigt sein, In Kohlenstoffketten oder-ringen können ein oder mehrere, zum Beispiel 1, 2, 3, 4, 5, 6, 7, 8, 9, 10, 11, 12 oder 13 Wasserstoffatome durch Fluoratome substituiert sein. Substituierte Kohlenstoffketten können in beliebigen Positionen substituiert sein.

Alkylreste können geradkettig oder verzweigt sein. Dies gilt auch, wenn sie Substituenten tragen oder als Substituenten anderer Reste auftreten, beispielsweise in Fluoralkylresten oder Alkoxyresten. Beispiele für Alkylreste sind Methyl, Ethyl, n-Propyl, Isopropyl (= 1-Methylethyl), n-Butyl, Isobutyl (= 2-Methylpropyl), sec-Butyl (= 1-Methylpropyl), tert-Butyl (= 1,1-Dimethylethyl), n-Pentyl, Isopentyl, tert-Pentyl, Neopentyl, n-Hexyl, 3-Methylpentyl, Isohexyl, Neohexyl. Bevorzugte Alkylreste sind Methyl, Ethyl, Isopropyl. In Alkylresten können ein oder mehrere, zum Beispiel 1, 2, 3, 4, 5, 6, 7, 8, 9, 10, 11, 12 oder 13, Wasserstoffatome durch Fluoratome substituiert sein. Beispiele für solche Fluoralkylreste sind Trifluormethyl, 2,2,2-Trifluorethyl, Pentafluorethyl, Heptafluorisopropyl. Substituierte Alkylreste können in beliebigen Positionen substituiert sein.

Alkenylreste können geradkettig oder verzweigt sein. Dies gilt auch, wenn sie Substituenten tragen beispielsweise in Fluoralkenylresten. Die Alkenylreste können in unterschiedlichen Positionen ungesättigt und auch mehrfach ungesättigt sein. Beispiele für Alkenylreste sind Ethenyl, n-Prop-1-enyl, n-Prop-2-enyl, Isoprop-1-enyl (= 1-Methylethenyl), n-But-1-enyl, n-But-2-enyl, n-But-3-enyl, n-Buta-1,3-dienyl, Isobut-1-enyl (= 2-Methylprop-1-enyl), Isobut-2-enyl (= 2-Methylprop-2-enyl), sec-But-1-enyl (= 1-Methylprop-1-enyl), Pentenyl, Hexenyl. Bevorzugte Alkenylreste sind Ethenyl, n-Prop-1-enyl, n-Prop-2-enyl, n-But-1-enyl, n-But-2-enyl, n-Pentenyl, n-Pentadienyl, Isopentenyl, tert-Pentenyl, Neopentenyl, n-Hexenyl, n-Hexadienyl, n-Hexatrienyl, 3-Methylpentenyl, Isohexenyl, Neohexenyl. In Alkenylresten können ein oder mehrere, zum Beispiel 1, 2, 3, 4, 5, 6, 7, 8, 9, 10 oder 11 Wasserstoffatome durch Fluoratome substituiert sein. Substituierte Alkenylreste können in beliebigen Positionen substituiert sein.

Alkinylreste können geradkettig oder verzweigt sein. Dies gilt auch, wenn sie Substituenten tragen beispielsweise in Fluoralkinylresten. Die Alkinylreste können in unterschiedlichen Positionen ungesättigt und auch mehrfach ungesättigt sein. Beispiele für Alkinylreste sind Ethinyl, n-Prop-1-inyl, n-Prop-2-inyl, n-But-1-inyl, n-But-2-inyl, n-But-3-inyl, n-Buta-1,3-diinyl, sec-But-2-inyl (= 1-Methylprop-2-inyl), n-Pentinyl, n-Pentadiinyl, Isopentinyl, tert-Pentinyl, Neopentinyl, n-Hexinyl, n-Hexadiinyl, n-Hexatriinyl, 3-Methylpentinyl, Isohexinyl, Neohexinyl. Bevorzugte Alkinylreste sind n-Prop-1-inyl, n-Prop-2-inyl, n-But-1-inyl, n-But-2-inyl. In Alkinylresten können ein oder mehrere, zum Beispiel 1, 2, 3, 4, 5, 6, 7, 8 oder 9, Wasserstoffatome durch Fluoratome substituiert sein. Substituierte Alkinylreste können in beliebigen Positionen substituiert sein.

Beispiele für Cycloalkylreste sind Cyclopropyl, Cyclobutyl, Cyclopentyl oder Cyclohexyl. In Cycloalkylresten können ein oder mehrere, zum Beispiel 1, 2, 3, 4, 5, 6, 7, 8, 9, 10 oder 11 Wasserstoffatome durch Fluoratome substituiert sein. Substituierte Cycloalkylreste können in beliebigen Positionen substituiert sein.

Die Cycloalkenylreste können in unterschiedlichen Positionen ungesättigt und auch mehrfach ungesättigt sein. Beispiele für Cycloalkenylreste sind Cyclobut-1-enyl, Cyclobut-2-enyl, Cyclopentenyl, Cyclopentadienyl, Cyclohexenyl oder Cyclohexadienyl. In Cycloalkenylresten können ein oder mehrere, zum Beispiel 1, 2, 3, 4, 5, 6, 7, 8 oder 9 Wasserstoffatome durch Fluoratome substituiert sein. Substituierte Cycloalkenylreste können in beliebigen Positionen substituiert sein.

Phenylreste können unsubstituiert sein oder einfach oder mehrfach, zum Beispiel einfach, zweifach oder dreifach, durch gleiche oder verschiedene Reste substituiert sein. In monosubstituierten Phenylresten kann sich der Substituent in der 2-Position, der 3-Position oder der 4-Position befinden. Zweifach substituiertes Phenyl kann in 2,3-Position, 2,4-Position, 2,5-Position, 2,6-Position, 3,4-Position oder 3,5-Position substituiert sein.

Beschrieben wird auch eine Methode zur Herstellung der verwendeten Verbindungen. So lassen sich die durch Formel I beschriebenen Verbindungen in dem Fachmann bekannter Weise unter sauren Bedingungen aus den zugrundeliegenden Guanidinen der Formel II oder einer tautomeren Form der Formel II darstellen, 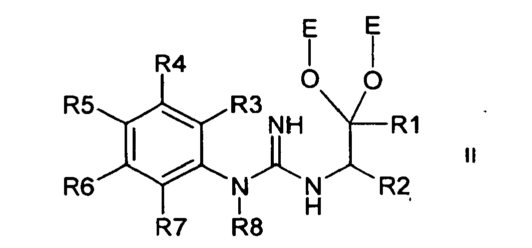 wobei die Reste R1 bis R8 entsprechend Formel I definiert sind und E einem Alkylrest mit 1 bis 4 Kohlenstoffatomen entspricht, wobei die beiden Reste E auch zu einem Ring verbunden sein können.

Verbindungen der Formel II können in literaturbekannter Weise aus den Cyanamiden der Formel III oder Verbindungen der Formel IV und den entsprechenden Aminoketalen oder -acetalen der Formel V erhalten werden, wobei der Rest R in Formel IV einer Alkylgruppe, bevorzugt Methyl, entspricht.

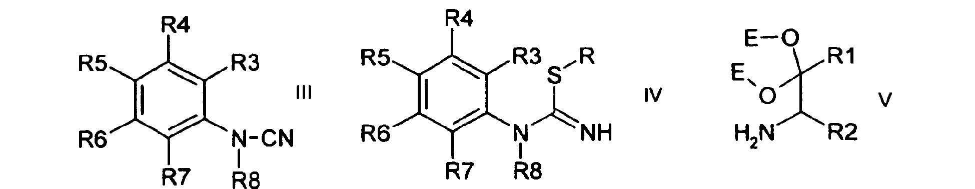

Der Zugang zu den obigen Intermediaten der Formel III, IV und V für R8 = Wasserstoff ist literaturbekannt (J. Med. Chem. 1975, 18, 90-99; GB1,131,191). Es wurde gefunden, dass Verbindungen der Formel V sich in Abwandlung obiger Literatur ausgehend von α-Haloketonen oder -aldehyden vorteilhaft über die Sequenz Halogen-Azid-Austausch (bevorzugt mit Natriumazid), Ketalisierung oder Acetalisierung (bevorzugt mit Ethylenglykol) und Reduktion (bevorzugt mit Wasserstoff in Gegenwart von Palladium auf Kohle oder Platindioxid) darstellen lassen (Schema 1).

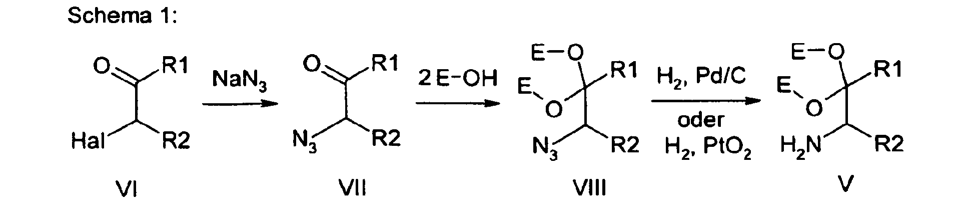

Die vorliegende Erfindung betrifft somit ferner ein Verfahren zur Herstellung von α-Aminoketalen der Formel (V), 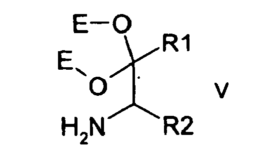 worin bedeuten:
- R1 und R2: zusammen mit den beiden Kohlenstoffatomen, an die sie gebunden sind, einen fünf- bis achtgliedrigen gesättigten oder eine Doppelbindung enthaltenden Kohlenstoffring,
wobei der Ring unsubstituiert oder mit 1, 2, 3, 4, 5, 6, 7, 8, 9, 10, 11 oder 12 F-Atomen und/oder mit ein oder zwei Resten aus der Gruppe NR11 R12, Alkyl mit 1, 2, 3 oder 4 C-Atomen, CN, CF₃ oder Alkoxy mit 1, 2, 3 oder 4 C-Atomen substituiert ist mit
R11, R12 unabhängig voneinander Alkyl mit 1, 2, 3 oder 4 C-Atomen, Benzyl, 4-Methoxybenzyl;
- E: Alkyl mit 1, 2, 3 oder 4 C-Atomen oder beide Reste E ein cyclisches Ketal ausbilden, in dem E-E (C₂- C₄)-Alkylen ist;
- Hal: Cl, Br, I
dadurch gekennzeichnet,
dass ein α-Halogenketon oder -aldehyd der Formel VI mit einem Azid, zum Beispiel NaN₃, unter Halogen-Azid-Austausch in das entsprechende α-Azidketon oder -aldehyd der Formel VII überführt wird, 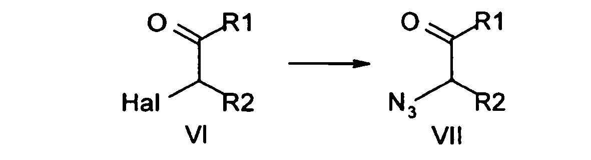 das anschließend mit ein- oder zweiwertigen Alkoholen unter Ketalisierung oder Acetalisierung zum α-Azidketal oder -acetal der Formel VIII umgesetzt wird, 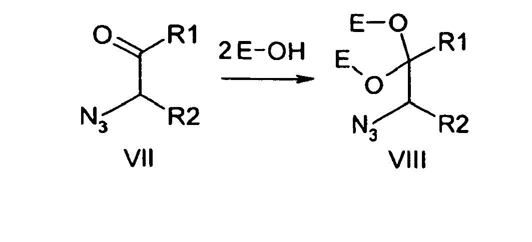 2,3-Position, 2,4-Position, 2,5-Position, 2,6-Position, 3,4-Position oder 3,5-Position substituiert sein.

Beschrieben wird auch eine Methode zur Herstellung der verwendeten Verbindungen. So lassen sich die durch Formel I beschriebenen Verbindungen in dem Fachmann bekannter Weise unter sauren Bedingungen aus den zugrundeliegenden Guanidinen der Formel II oder einer tautomeren Form der Formel II darstellen, 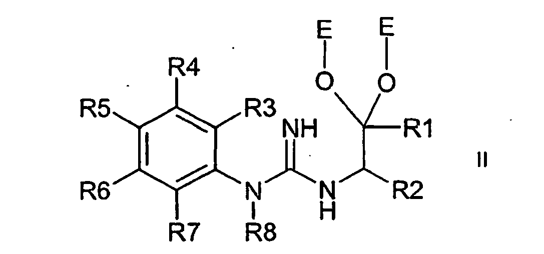 wobei die Reste R1 bis R8 entsprechend Formel I definiert sind und E einem Alkylrest mit 1 bis 4 Kohlenstoffatomen entspricht, wobei die beiden Reste E auch zu einem Ring verbunden sein können.

Verbindungen der Formel II können in literaturbekannter Weise aus den Cyanamiden der Formel III oder Verbindungen der Formel IV und den entsprechenden Aminoketalen oder -acetalen der Formel V erhalten werden, wobei der Rest R in Formel IV einer Alkylgruppe, bevorzugt Methyl, entspricht.

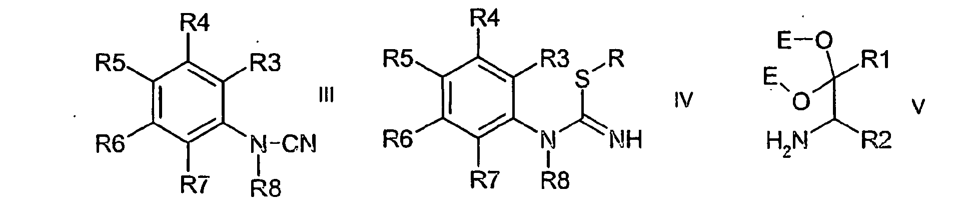

Der Zugang zu den obigen Intermediaten der Formel III, IV und V für R8 = Wasserstoff ist literaturbekannt (J. Med. Chem. 1975, 18, 90-99; GB1,131,191). Es wurde gefunden, dass Verbindungen der Formel V sich in Abwandlung obiger Literatur ausgehend von α-Haloketonen oder -aldehyden vorteilhaft über die Sequenz Halogen-Azid-Austausch (bevorzugt mit Natriumazid), Ketalisierung oder Acetalisierung (bevorzugt mit Ethylenglykol) und Reduktion (bevorzugt mit Wasserstoff in Gegenwart von Palladium auf Kohle oder Platindioxid) darstellen lassen (Schema 1).

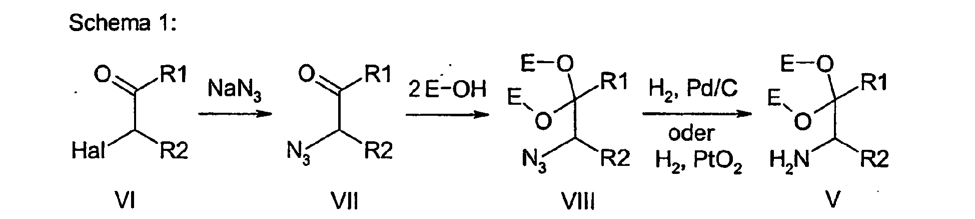

Die vorliegende Erfindung betrifft somit ferner ein Verfahren zur Herstellung von α-Aminoketalen der Formel (V), 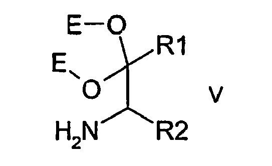 worin bedeuten:
- R1 und R2: H, Alkyl mit 1, 2, 3, 4, 5 oder 6 C-Atomen, Alkenyl mit 2, 3, 4, 5 oder 6 C-Atomen, Alkinyl mit 2, 3, 4, 5 oder 6 C-Atomen, Cycloalkyl mit 3, 4, 5 oder 6 C-Atomen, Cycloalkenyl mit 4, 5 oder 6 C-Atomen oder Phenyl,
wobei Phenyl unsubstituiert oder unabhängig voneinander mit ein oder zwei Resten aus der Gruppe F, Cl, Br, l, NR11R12, Alkyl mit 1, 2, 3 oder 4 C-Atomen, CN, CF₃ oder Alkoxy mit 1, 2, 3 oder 4 C-Atomen substituiert ist mit
R11, R12 unabhängig voneinander Alkyl mit 1, 2, 3 oder 4 C-Atomen, Benzyl, 4-Methoxybenzyl; und
wobei die Kohlenstoffketten unsubstituiert sind oder unabhängig voneinander mit 1, 2, 3, 4, 5, 6, 7, 8 oder 9 F-Atomen und/oder mit ein oder zwei Resten aus der Gruppe NR11 R12, Alkyl mit 1, 2, 3 oder 4 C-Atomen, CN, CF₃ oder Alkoxy mit 1, 2, 3 oder 4 C-Atomen substituiert sind mit
R11, R12 unabhängig voneinander Alkyl mit 1, 2, 3 oder 4 C-Atomen, Benzyl, 4-Methoxybenzyl; oder
- R1 und R2: zusammen mit den beiden Kohlenstoffatomen, an die sie gebunden sind, einen fünf- bis achtgliedrigen gesättigten oder eine Doppelbindung enthaltenden Kohlenstoffring,
wobei der Ring unsubstituiert oder mit 1, 2, 3, 4, 5, 6, 7, 8, 9, 10, 11 oder 12 F-Atomen und/oder mit ein oder zwei Resten aus der Gruppe NR11 R12, Alkyl mit 1, 2, 3 oder 4 C-Atomen, CN, CF₃ oder Alkoxy mit 1, 2, 3 oder 4 C-Atomen substituiert ist mit
R11, R12 unabhängig voneinander Alkyl mit 1, 2, 3 oder 4 C-Atomen, Benzyl, 4-Methoxybenzyl;
- E: Alkyl mit 1, 2, 3 oder 4 C-Atomen oder beide Reste E ein cyclisches Ketal ausbilden, in dem E-E (C₂- C₄)-Alkylen ist;
- Hal: Cl, Br, I
dadurch gekennzeichnet,
dass ein α-Halogenketon oder -aldehyd der Formel VI mit einem Azid, zum Beispiel NaN₃, unter Halogen-Azid-Austausch in das entsprechende α-Azidketon oder -aldehyd der Formel VII überführt wird, 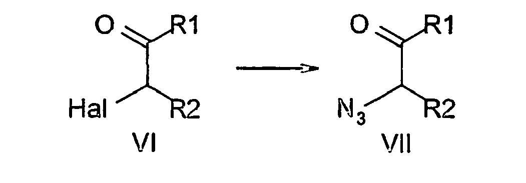 das anschließend mit ein- oder zweiwertigen Alkoholen unter Ketalisierung oder Acetalisierung zum α-Azidketal oder-acetal der Formel VIII umgesetzt wird, 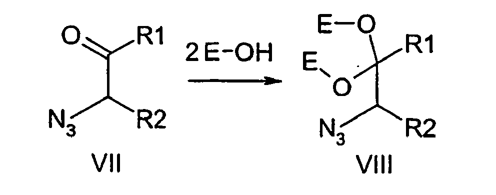 welches dann, bevorzugt mit Wasserstoff in Gegenwart eines Katalysators, zum Beispiel Palladium auf Kohle oder Platindioxid, zu Verbindungen der Formel V reduziert wird.

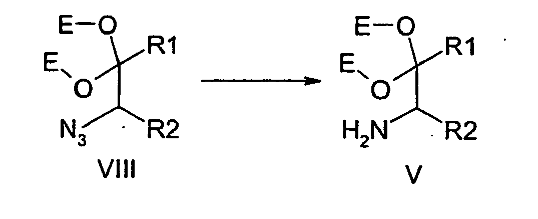

Weiterhin lassen sich Verbindungen der Formel I ausgehend von Cyanamiden der Formel III und α-Aminoketonen der Formel IX in protischen Solvenzien wie Alkohlen, zum Beispiel Ethanol, darstellen.

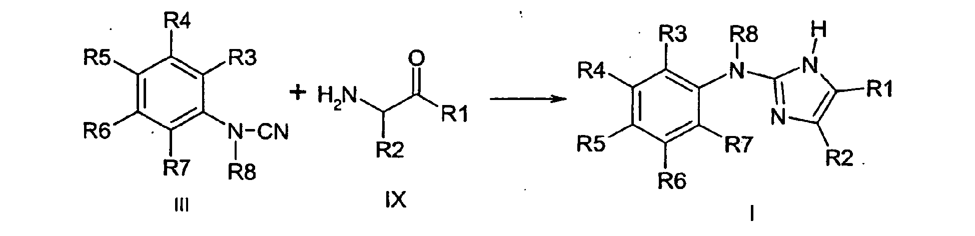

Alternativ kommt auch die zweistufige Umsetzung geeigneter 1,2-Diaminoalkylenderivate der Formel X mit Isothiocyanaten der Formel in XI in Frage.

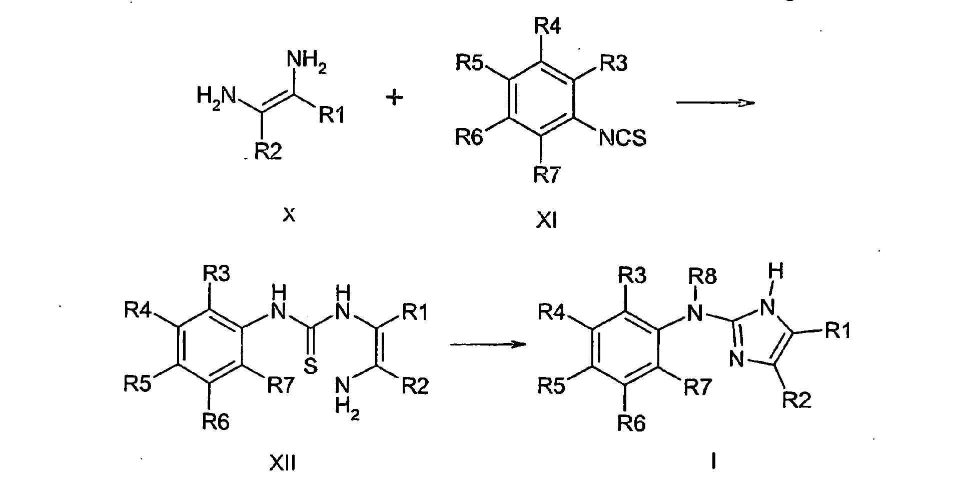

Der intermediär gebildete Thioharnstoff der Formel XII wird dann bevorzugt mit N,N'-Dicyclohexylcarbodiimid oder Methyliodid zu Verbindungen der Formel I cyclisiert.

Die Ausgangsverbindungen der Formeln IX und X sind käuflich erhältlich oder können nach oder analog zu in der Literatur beschriebenen, dem Fachmann bekannten Verfahren hergestellt werden.

Bei der vorliegenden Erfindung konnte überraschend gezeigt werden, dass die beschriebenen Verbindungen potente Inhibitoren des Natrium/ Wasserstoff-Austauschers (NHE) darstellen, insbesondere des NHE3.

Der NHE3 wird im Körper verschiedener Spezies bevorzugt in der Galle, dem Darm und in der Niere gefunden (Fliegel et al, Biochem. Cell. Biol. 76: 735 - 741, 1998), ließ sich aber auch im Gehirn nachweisen (E. Ma et al. Neuroscience 79: 591 - 603).

Bekannte NHE3-Inhibitoren leiten sich beispielsweise von Verbindungen des Acylguanidin-Typs (EP 825 178), Norbornylamin-Typs (DE 199 60 204), 2-Guanidinochinazolin-Typs (WO 01 79 186, WO 02 20496) oder Benzamidin-Typs (WO 01 21582, WO 01 72 742) ab. Das ebenfalls als NHE3-Inhibitor beschriebene Squalamin (M. Donowitz et al. Am. J. Physiol. 276 (Cell Physiol. 45: C136 - C144) wirkt nicht unmittelbar wie die Verbindungen nach Formel I, sondern erreicht seine maximale Wirkstärke erst nach einer Stunde.

In der deutschen Patentanmeldung DE 10163239 werden NHE3-Inhibitoren vom Imidazolidin-Typ beschrieben, in der deutschen Patentanmeldung DE 10224892 vom Thiophen-Typ. NHE3-Inhibitoren vom 2-Phenylamino-benzimidazol-Typ werden in WO 02 46169 A1 beschrieben. Überraschend wurde nun gefunden, dass die hier beschriebenen Imidazol-Derivate der Formel I ebenfalls potente Inhibitoren des NHE3 darstellen und dabei vorteilhafte pharmakologische Eigenschaften besitzen.

Das den hier beschriebenen Verbindungen ähnliche Clonidin ist als schwacher NHE-Inhibitor bekannt. Allerdings ist seine Wirkung auf den NHE3 der Ratte mit einer IC₅₀ von 620 µM äußerst moderat. Stattdessen weist es eine gewisse Selektivität für den NHE2 auf, an dem es eine I1C₅₀ von 42 µM besitzt (J. Orlowski et al J. Biol. Chem. 268, 25536). Es ist daher eher als NHE2-Inhibitor zu bezeichnen. Neben der schwachen NHE-Wirkung besitzt das Clonidin eine hohe Affinität zum adrenergen α2-Rezeptor und Imidazolin l1-Rezeptor wodurch eine starke blutdrucksenkende Wirkung vermittelt wird (Ernsberger et al Eur. J. Pharmacol. 134, 1, 1987). Verbindungen der Formel I zeichnen sich gegenüber Clonidin durch gesteigerte NHE3-inhibierende Wirkung aus.

Aufgrund dieser unerwarteten NHE-inhibitorischen Eigenschaften eignen sich die Verbindungen der Formel I zur Prävention und Behandlung von Krankheiten, die durch eine Aktivierung bzw. durch einen aktivierten NHE verursacht werden. Die Verwendung der erfindungsgemäßen Verbindungen betrifft die Prävention und die Behandlung akuter und chronischer Krankheiten in der Veterinär und in der Humanmedizin.

So eignen sich die erfindungsgemäßen Inhibitoren des NHE zur Behandlung von Krankheiten, die durch Ischämie und / oder durch Reperfusion hervorgerufen werden.

Die hier beschriebenen Verbindungen sind infolge ihrer pharmakologischen Eigenschaften als antiarrhythmische Arzneimittel mit cardioprotektiver Komponente zur Infarktprophylaxe und der Infarktbehandlung sowie zur Behandlung der angina pectoris hervorragend geeignet, wobei sie auch präventiv die pathophysiologischen Vorgänge beim Entstehen ischämisch induzierter Schäden, insbesondere bei der Auslösung ischämisch induzierter Herzarrhythmien, inhibieren oder stark vermindern. Wegen ihrer schützenden Wirkungen gegen pathologische hypoxische und ischämische Situationen können die erfindungsgemäß verwendeten Verbindungen der Formel I infolge Inhibition des zellulären Na⁺/H⁺-Austauschmechanismus als Arzneimittel zur Behandlung aller akuten oder chronischen durch Ischämie ausgelösten Schäden oder dadurch primär oder sekundär induzierten Krankheiten verwendet werden. Dies betrifft ihre Verwendung als Arzneimittel für operative Eingriffe, z.B. bei Organ-Transplantationen, wobei die Verbindungen sowohl für den Schutz der Organe im Spender vor und während der Entnahme, zum Schutz entnommener Organe beispielsweise bei Behandlung mit oder deren Lagerung in physiologischen Badflüssigkeiten, wie auch bei der Überführung in den Empfängerorganismus verwendet werden können. Die Verbindungen sind ebenfalls wertvolle, protektiv wirkende Arzneimittel bei der Durchführung angioplastischer operativer Eingriffe beispielsweise am Herzen wie auch an peripheren Gefäßen.

Entsprechend ihrer protektiven Wirkung gegen ischämisch induzierte Schäden sind die Verbindungen auch als Arzneimittel zur Behandlung von Ischämien des Nervensystems, insbesondere des ZNS, geeignet, wobei sie z.B. zur Behandlung des Schlaganfalls oder des Himödems geeignet sind.

Darüber hinaus eignen sich die erfindungsgemäß verwendeten Verbindungen der Formel I ebenfalls zur Behandlung von Formen des Schocks, wie beispielweise des allergischen, cardiogenen, hypovolämischen und des bakteriellen Schocks.

Weiterhin induzieren die Verbindungen eine Verbesserung des Atemantriebes und werden deshalb zur Behandlung von Atmungszuständen bei folgenden klinischen Zuständen und Krankheiten herangezogen: Gestörter zentraler Atemantrieb (z. B. zentrale Schlafapnoen, plötzlicher Kindstod, postoperative Hypoxie), muskulärbedingte Atemstörungen, Atemstörungen nach Langzeitbeatmung, Atemstörungen bei Adaptation im Hochgebirge, obstruktive und gemischte Form der Schlafapnoen, akute und chronische Lungenkrankheiten mit Hypoxie und Hyperkapnie.
Zusätzlich erhöhen die Verbindungen den Muskeltonus der oberen Atemwege, so dass das Schnarchen unterdrückt wird.
Eine Kombination eines NHE-Inhibitors mit einem Carboanhydrase-Hemmer (z. B. Acetazolamid), wobei letzterer eine metabolische Azidose herbeiführt und dadurch bereits die Atmungstätigkeit steigert, erweist sich als vorteilhaft durch verstärkte Wirkung und verminderten Wirkstoffeinsatz.

Außerdem eignen sich die hier beschriebenen Verbindungen als Medikamente zur Therapie und Prophylaxe von Erkrankungen und Störungen, die durch Übererregbarkeit des Zentralnervensystems ausgelöst werden, insbesondere zur Behandlung von Erkrankungen des epileptischen Formenkreises, zentral ausgelösten klonischen und tonischen Spasmen, psychischen Depressionszuständen, Angsterkrankungen und Psychosen. Dabei können die hier beschriebenen NHE-Inhibitoren allein angewandt werden oder in Kombination mit anderen antiepileptisch wirkenden Substanzen oder antipsychotischen Wirkstoffen, oder Carboanhydratasehemmern, beispielweise mit Acetazolamid, sowie mit weiteren Inhibitoren des NHE oder des Natrium-abhängigen Chlorid-Bicarbonat-Austauschers (NCBE).

Es hat sich gezeigt, dass die erfindungsgemäß verwendeten Verbindungen eine milde abführende Wirkung besitzen und demzufolge vorteilhaft als Abführmittel oder bei drohender Darmverstopfung verwendet werden können.

Außerdem können die erfindungsgemäßen Verbindungen vorteilhaft zur Prävention und Therapie von akuten und chronischen Erkrankungen des Darmtrakts verwendet werden, die durch ischämische Zustände im Intestinalbereich und / oder durch nachfolgende Reperfusion ausgelöst werden. Solche Komplikationen können beispielweise durch mangelnde Darmperistaltik hervorgerufen werden, wie sie z.B. häufig nach chirurgischen Eingriffen, bei Darmverstopfung oder stark verminderter Darmtätigkeit zu beobachten sind.
Weiterhin besteht die Möglichkeit, der Gallenstein-Bildung vorzubeugen.

Darüber hinaus zeichnen sich die erfindungsgemäß verwendeten Verbindungen der Formel I durch starke inhibierende Wirkung auf die Proliferation von Zellen, beispielsweise der Fibroblasten-Zellproliferation und der Proliferation der glatten Gefäßmuskelzellen, aus. Deshalb kommen die Verbindungen der Formel I als wertvolle Therapeutika für Krankheiten in Frage, bei denen die Zellproliferation eine primäre oder sekundäre Ursache darstellt, und können deshalb als Antiatherosklerotika, Mittel gegen diabetische Spätkomplikationen, Mittel gegen das chronische Nierenversagen, Krebserkrankungen, fibrotische Erkrankungen des Herzens, sowie Lungenfibrose, Leberfibrose oder Nierenfibrose, Organhypertrophien und -hyperplasien, beispielsweise des Herzens und der Prostata verwendet werden, und somit zur Prävention und Behandlung der Herzinsuffizienz (congestive heart failure) oder bei Prostatahyperplasie bzw. Prostatahypertrophie benutzt werden.

Die erfindungsgemäßen Verbindungen sind wirkungsvolle Inhibitoren des zellulären Natrium-Protonen-Antiporters (Na/H-Exchanger), der bei zahlreichen Erkrankungen (Essentielle Hypertonie, Atherosklerose, Diabetes usw.) auch in solchen Zellen erhöht ist, die Messungen leicht zugänglich sind, wie beispielsweise in Erythrocyten, Thrombocyten oder Leukozyten, Die erfindungsgemäß verwendeten Verbindungen eignen sich deshalb als hervorragende und einfache wissenschaftliche Werkzeuge, beispielsweise in ihrer Verwendung als Diagnostika zur Bestimmung und Unterscheidung bestimmter Formen der Hypertonie, aber auch der Atherosklerose, des Diabetes und diabetischer Spätkomplikationen, proliferativer Erkrankungen usw.

Darüber hinaus sind die Verbindungen der Formel I für die präventive Therapie zur Verhinderung der Genese und zur Behandlung des Bluthochdrucks, beispielweise der essentiellen Hypertonie, geeignet, da sie die Rückresorption von NaCl im tubulären System der Niere vermindern oder vollständig inhibieren. Entsprechend sind sie auch hervorragend als Kombinations- und Formulierungspartner für Arzneimittel geeignet, die zur Bluthochdruckbehandlung verwendet werden. So können beispielsweise thiazid-artig wirkende Diuretika, Loop-Diuretika, Aldosteron- und Pseudoaldosteron-Antagonisten, wie Hydrochlorothiazid, Indapamid, Polythiazid, Furosemid, Piretanid, Torasemid, Bumetanid, Amilorid, Triamteren kombiniert werden. Weiterhin können die NHE-Inhibitoren der vorliegenden Erfindung in Kombination mit ACE-Hemmem, wie beispielsweise Ramipril, Enalapril oder Captopril verwendet werden. Weitere günstige Kombinationspartner sind auch β-Blocker.

Die beschriebenen NHE-Inhibitoren können ebenfalls in der Prävention und zur Behandlung thrombotischer Erkrankungen verwendet werden, da sie als NHE-Inhibitoren sowohl die Plättchenaggregation selbst inhibieren können und darüber hinaus die überschießende Freisetzung von Gerinnungsmediatoren, insbesondere des von Willebrand Faktors hemmen bzw. verhindern können. Deshalb sind die NHE-Inhibitoren der vorliegenden Erfindung kombinierbar mit weiteren antikoagulativen Wirkstoffen wie beispielsweise Acetylsalizylsäure, Thrombinantagonisten, Faktor Xa-Antagonisten, fibrinolytisch wirkenden Arzneistoffen, Faktor-VIIa-Antagonisten usw. Eine kombinierte Anwendung der vorliegenden NHE-Inhibitoren mit NCBE-Inhibitoren ist besonders günstig.
Es wurde außerdem gefunden, dass NHE-Inhibitoren eine günstige Beeinflussung der Serumlipoproteine zeigen. Es ist allgemein anerkannt, dass für die Entstehung arteriosklerotischer Gefäßveränderungen, insbesondere der koronaren Herzkrankheit, zu hohe Blutfettwerte, sogenannte Hyperlipoproteinämien, einen wesentlichen Risikofaktor darstellen. Für die Prophylaxe und die Regression von atherosklerotischen Veränderungen kommt daher der Senkung erhöhter SerumLipoproteine eine außerordentliche Bedeutung zu. Die erfindungsgemäß verwendeten Verbindungen können daher zur Prophylaxe und zur Regression von atherosklerotischen Veränderungen herangezogen werden, indem sie einen kausalen Risikofaktor ausschalten. Die erfindungsgemäßen Inhibitoren des NHE können in günstiger Weise auch mit anderen antiarteriosklerotischen Wirkstoffen kombiniert werden, wie einem Stoff aus der Klasse der Fibrate, einem Upregulator der LD2 Rezeptoraktivität, wie MD-700 und LY295427, oder einem Cholesterol- oder Gallensäure-Resorptionshemmer oder einem Antihyperchlesterolämikum aus der Klasse der Statine, wie beispielsweise Pravastatin, Lovastatin, Simvastatin.

Mit diesem Schutz der Gefäße gegen das Syndrom der endothelialen Dysfunktion sind Verbindungen der Formel I wertvolle Arzneimittel zur Prävention und zur Behandlung koronarer Gefäßspasmen, peripherer Gefäßkrankheiten, wie claudicatio intermittens, der Atherogenese und der Atherosklerose, der linksventrikulären Hypertrophie und der dilatierten Kardiomyopathie, und thrombotischer Erkrankungen.

Die genannten Verbindungen können ebenfalls zur Behandlung von Krankheiten verwendet werden, die durch Protozoen verursacht werden und eignen sich insbesondere als Antimalariamittel. Darüber hinaus eignen sich die Verbindungen zur Bekämpfung saugender Parasiten, wie Moskitos, Zecken, Flöhen und Pflanzenschädlingen.

Entsprechend ihrer protektiven Wirkungen sind die Verbindungen auch als Arzneimittel zur Gesunderhaltung und Lebensverlängerung geeignet.

Generell können die hier beschriebenen NHE-Inhibitoren in günstiger Weise mit anderen, den intrazellulären pH-regulierenden Verbindungen kombiniert werden,
wobei Inhibitoren der Enzymgruppe der Carboanhydratase, Inhibitoren der Bicarbonationen transportierenden Systeme wie des Natrium-Bicarbonat-Cotransporters oder des Natrium abhängigen Chlorid-Bicarbonat-Austauschers, sowie andere NHE-Inhibitoren, beispielweise mit inhibitorischer Wirkung auf andere NHE-Subtypen, als Kombinationspartner infrage kommen, weil durch sie die pharmakologisch relevanten pH-regulierenden Effekte der hier beschriebenen NHE-inhibitoren verstärkt werden können.

Die genannten Verbindungen finden deshalb vorteilhaft Verwendung zur Herstellung eines Medikaments zur Prävention und Behandlung von Schlafapnoen und muskulär bedingter Atemstörungen; zur Herstellung eines Medikaments zur Prävention und Behandlung des Schnarchens; zur Herstellung eines Medikaments zur Blutdrucksenkung; zur Herstellung eines Medikaments mit abführender Wirkung zur Prävention und Behandlung intestinaler Verstopfungen; zur Herstellung eines Medikaments zur Prävention und Behandlung von Erkrankungen, die durch Ischämie und Reperfusion von zentralen und peripheren Organen ausgelöst werden wie das akute Nierenversagen, der Schlaganfall, endogene Schockzustände, Darmerkrankungen etc.; zur Herstellung eines Medikamentes zur Behandlung diabetischer Spätschäden und chronischer Nierenerkrankungen, insbesondere von allen Nierenentzündungen (Nephritiden), die mit einer vermehrten Protein/Albuminausscheidung verbunden sind; zur Herstellung eines Medikaments zur Behandlung von Hypercholesterinämie; zur Herstellung eines Medikaments zur Prävention der Atherogenese und der Atherosklerose; zur Herstellung eines Medikaments zur Prävention und Behandlung von Krankheiten, die durch erhöhte Cholesterinspiegel ausgelöst werden; zur Herstellung eines Medikaments zur Prävention und Behandlung von Krankheiten, die durch endotheliale Dysfunktion ausgelöst werden; zur Herstellung eines Medikaments zur Behandlung des Befalls durch Ektoparasiten; zur Herstellung eines Medikaments zur Behandlung der genannten Leiden in Kombinationen mit blutdrucksenkenden Stoffen, bevorzugt mit Angiotensin Converting Enzyme (ACE)-Hemmern, mit Diuretika, Aldosteron-Antagonisten und Angiotensin-Rezeptorantagonisten. Eine Kombination eines NHE-Inhibitors der Formel I mit einem blutfettspiegelsenkenden Wirkstoff, bevorzugt mit einem HMG-CoA-Reduktaseinhibitor (z. B. Lovastatin oder Pravastatin), wobei letzterer eine hypolipidämische Wirkung herbeiführt und dadurch die hypolipidämischen Eigenschaften des NHE-Inhibitors der Formel steigert, erweist sich als günstige Kombination mit verstärkter Wirkung und vermindertem Wirkstoffeinsatz.

Die Erfindung betrifft auch Heilmittel für die humane, veterinäre oder phytoprotektive Anwendung enthaltend eine wirksame Menge einer Verbindung der Formel I und / oder eines pharmazeutisch verträglichen Salzes davon, ebenso wie Heilmittel für die humane, veterinäre oder phytoprotektive Anwendung enthaltend eine wirksame Menge einer Verbindung der Formel I und / oder eines pharmazeutisch verträglichen Salzes davon allein oder in Kombination mit einem oder mehreren anderen pharmakologischen Wirkstoffen oder Arzneimitteln.

Arzneimittel, die eine Verbindung der Formel I enthalten, können dabei zum Beispiel oral, parenteral, intravenös, rektal, topisch oder durch Inhalation appliziert werden,
wobei die bevorzugte Applikation von dem jeweiligen Erscheinungsbild der Erkrankung abhängig ist. Die Verbindungen der Formel I können dabei allein oder zusammen mit galenischen Hilfsstoffen zur Anwendung kommen, und zwar sowohl in der Veterinärals auch in der Humanmedizin und im Pflänzenschutz.

Welche Hilfsstoffe für die gewünschte Arzneimittelformulierung geeignet sind, ist dem Fachmann auf Grund seines Fachwissens geläufig. Neben Lösemitteln, Gelbildnern, Suppositorien-Grundlagen, Tablettenhilfsstoffen, und anderen Wirkstoffträgern können beispielsweise Antioxidantien, Dispergiermittel, Emulgatoren, Entschäumer, Geschmackskorrigentien, Konservierungsmittel, Lösungsvermittler oder Farbstoffe verwendet werden.

Für eine orale Anwendungsform werden die aktiven Verbindungen mit den dafür geeigneten Zusatzstoffen, wie Trägerstoffen, Stabilisatoren oder inerten Verdünnungsmittel vermischt und durch die üblichen Methoden in die geeigneten Darreichungsformen gebracht, wie Tabletten, Dragees, Steckkapseln, wässrige, alkoholische oder ölige Lösungen. Als inerte Träger können z. B. Gummi arabicum, Magnesia, Magnesiumcarbonat, Kaliumphosphat, Milchzucker, Glucose oder Stärke, insbesondere Maisstärke, verwendet werden. Dabei kann die Zubereitung sowohl als Trocken- als auch als Feuchtgranulat erfolgen. Als ölige Trägerstoffe oder als Lösemittel kommen beispielsweise pflanzliche oder tierische Öle in Betracht, wie Sonnenblumenöl oder Lebertran.

Zur subkutanen oder intravenösen Applikation werden die verwendeten aktiven Verbindungen, gewünschtenfalls mit den dafür üblichen Substanzen wie Lösungsvermittler, Emulgatoren oder weiteren Hilfsstoffen in Lösung, Suspension oder Emulsion gebracht. Als Lösungsmittel kommen z. B. in Frage: Wasser, physiologische Kochsalzlösung oder Alkohole, z. B. Ethanol, Propanol, Glycerin, daneben auch Zuckerlösungen wie Glucose- oder Mannitlösungen, oder auch eine Mischung aus den verschiedenen genannten Lösungsmitteln.

Als pharmazeutische Formulierung für die Verabreichung in Form von Aerosolen oder Sprays sind geeignet z. B. Lösungen, Suspensionen oder Emulsionen des Wirkstoffes der Formel l in einem pharmazeutisch unbedenklichen Lösungsmittel, wie insbesondere Ethanol oder Wasser, oder einem Gemisch solcher Lösungsmittel. Die Formulierung kann nach Bedarf auch noch andere pharmazeutische Hilfsstoffe wie Tenside, Emulgatoren und Stabilisatoren sowie ein Treibgas enthalten. Eine solche Zubereitung enthält den Wirkstoff üblicherweise in einer Konzentration von etwa 0,1 bis 10, insbesondere von etwa 0,3 bis 3 Gew.-%.

Die Dosierung des zu verabreichenden Wirkstoffs der Formel I und die Häufigkeit der Verabreichung hängen von der Wirkstärke und Wirkdauer der verwendeten Verbindungen ab; außerdem auch von Art und Stärke der zu behandelnden Krankheit sowie von Geschlecht, Alter, Gewicht und individueller Ansprechbarkeit des zu behandelnden Säugers.

Im Durchschnitt beträgt die tägliche Dosis einer Verbindung der Formel I bei einem etwa 75 kg schweren Patienten mindestens 0,001 mg/kg, vorzugsweise 0,1 mg/kg, bis höchstens 50 mg/kg, vorzugsweise 1 mg/kg Körpergewicht. Bei akuten Ausbrüchen der Krankheit, etwa unmittelbar nach Erleiden eines Herzinfarkts, können auch noch höhere und vor allem häufigere Dosierungen notwendig sein, z. B. bis zu 4 Einzeldosen pro Tag. Insbesondere bei i.v. Anwendung, etwa bei einem Infarktpatienten auf der Intensivstation, können bis zu 200 mg/kg pro Tag notwendig werden.

Pharmazeutisch verträgliche Salze werden z. B. über folgende Säuren hergestellt: aus anorganischen Säuren wie Salzsäure, Schwefelsäure oder Phosphorsäure oder aus organischen Säuren wie Essigsäure, Zitronensäure, Weinsäure, Milchsäure, Malonsäure, Methansulfonsäure, Fumarsäure. Als Säureadditionssalze kommen dabei Salze aller pharmakologisch verträglichen Säuren in Frage, beispielsweise Halogenide, insbesondere Hydrochloride, Lactate, Sulfate, Citrate, Tartrate, Acetate, Phosphate, Methylsulfonate, p-Toluolsulfonate, Adipinate, Fumarate, Gluconate, Glutamate, Glycerolphosphate, Maleinate und Pamoate (diese Gruppe entspricht auch den physiologisch akzeptablen Anionen); aber auch Trifluoracetate.

Versuchsbeschreibungen und Beispiele:

### Liste der verwendeten Abkürzungen:

- Rt: Retentionszeit
- TFA: Trifluoressigsäure
- LCMS: Liquid Chromatography Mass Spectroscopy
- MS: Mass Spectroscopy
- Cl: Chemical lonisation
- ES: Elektrospray

### Allgemeines:

Die im Folgenden angegebenen Retentionszeiten (Rₜ) beziehen sich auf LCMS-Messungen mit den folgenden Parametern:

### Methode A:

- stationäre Phase:: Merck Purospher, 3µ, 2 x 55 mm
- mobile Phase:: 95% H₂O (0,05% TFA) → 95% Acetonitril, 4 min; 95% Acetonitril,1,5 min; → 5% Acetonitril, 1 min; 0,5 ml/min.

### Methode B:

- stationäre Phase:: YMC J'sphere ODS H80, 2 x 33 mm
- mobile Phase:: 95% H₂O (0,05% TFA) → 95% Acetonitril, 2,3 min; 95% Acetonitril, 1 min; → 5% Acetonitril, 0,1 min; 1 ml/min.

### Methode C:

- stationäre Phase:: YMC J'sphere ODS H80, 2 x 33 mm
- mobile Phase:: 90% H₂O (0,05% TFA) → 95% Acetonitril, 2,5 min; 95% Acetonitril, 0,8 min; → 10% Acetonitril; 0,05 min; 1 ml/min.

### Methode D:

- stationäre Phase:: Merck Purospher, 3µ, 2 x 55 mm
- mobile Phase:: 95% H₂O (0,1% HCOOH) → 95% Acetonitril (0,1% HCOOH), 5 min; → 95% Acetonitril (0,1% HCOOH), 2 min; → 95% H₂O (0,1% HCOOH), 1 min; 0,45 ml/min.

### Methode E:

- stationäre Phase:: mobile Phase: YMC J'sphere ODS H80, 2 x 33 mm 98% H₂O (0,05% TFA)+2% Acetonitril, 0,3 min; 98% H₂O (0,05% TFA) → 95% Acetonitril, 2 min; 95% Acetonitril, 0,4 min; 1 ml/min.

Die präparative HPLC wurde unter folgenden Bedingungen durchgeführt:
- stationäre Phase:: Merck Purospher RP18 (10µM) 250 x 25 mm
- mobile Phase:: 90% H₂O (0,05% TFA) → 90% Acetonitril, 40 min; 25 ml/min

### Beispiel 1: nicht erfindungsgemäß (2,6-Dichlorphenyl)-(1H-imidazol-2-yl)-amin-Hydrochlorid

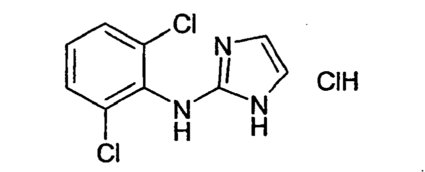

(2,6-Dichlorphenyl)-(1H-imidazol-2-yl)-amin-Hydrochlorid wurde nach literaturbekannter Methode hergestellt (J. Med. Chem., 1975, 18, 90-99; GB1131191). MS-Cl+: 228,1; LCMS-Rₜ (A)= 2,92 min

Beispiel 2: nicht erfindungsgemäß (2,6-Dichlorphenyl)-(4-methyl-1H-imidazol-2-yl)-amin-Hydrochlorid 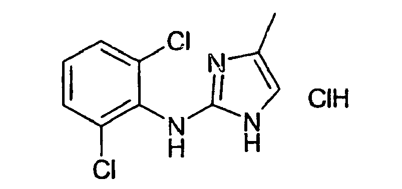
a) 2-(2-Oxo-propyl)-isoindol-1,3-dion 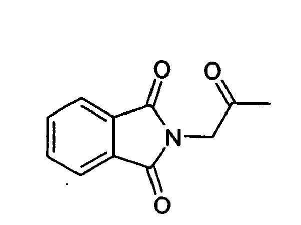
   Phthalimid (3 g) wurde in trockenem Tetrahydrofuran (70 ml) gelöst und bei Raumtemperatur zu einer Suspension aus Natriumhydrid (539 mg) in Tetrahydrofuran (15 ml) getropft. Anschließend wurde eine Stunde auf 40°C erwärmt und dann eine Lösung aus Chloraceton (1,99 g) gelöst in Tetrahydrofuran (15 ml) zugetropft. Anschließend wurde das Gemisch 15 Stunden auf Rückflusstemperatur gehalten. Nach Abkühlen wurde auf ein Eis/Wasser-Gemisch gegeben und dreimal mit Essigester extrahiert. Die vereinigten organischen Phasen wurden einmal mit gesättigter Natriumchlorid-Lösung gewaschen und dann über Magnesiumsulfat getrocknet. Das Lösungsmittel wurde im Vakuum entfernt und der Rückstand über Kieselgel (n-Heptan/Essigester 2:1) chromatographiert. Es wurden 2,9 g der Titelverbindung isoliert.
   MS-Cl+: 204,2; LCMS-Rₜ (C)= 1,60 min
b) C-(2-Methyl-[1,3]dioxolan-2-yl)-methylamin 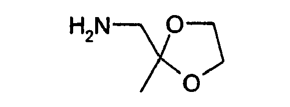 2-(2-Oxo-propyl)-isoindol-1,3-dion (2,9 g) wurde in Toluol (60 ml) mit Ethylenglykol (1,71 ml) und para-Toluolsulfonsäure (271 mg) versetzt und 15 Stunden am Wasserabscheider erhitzt. Anschließend wurde im Vakuum zur Trockne gebracht und der Rückstand in Ethanol (120 ml) gelöst. Nach Zugabe von Hydrazin (1 g) wurde eine Stunde auf 65°C erhitzt und dann ein weiteres Gramm Hydrazin zugegeben. Nach weiteren 1,5 Stunden bei 65 °C wurde das Heizen beendet. Nach Stehen über Nacht wurde der gebildete Niederschlag abgesaugt und das Filtrat im Vakuum eingeengt. Der Rückstand wurde in Ethanol aufgeschlämmt und erneut filtriert. Nach Einengen des Filtrats wurden 1,9 g des Amins erhalten, das direkt in der nächsten Stufe eingesetzt werden konnte.
   MS-Cl+: 118,2; LCMS-Rₜ (B)= 0,16 min
c) N-(2,6-Dichlorphenyl)-N'-(2-methyl-[1,3]dioxolan-2-ylmethyl)-guanidin-Trifluoressigsäuresalz 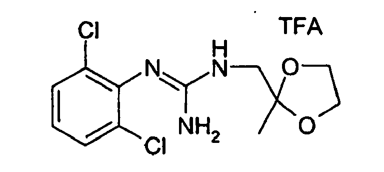 2,6-Dichlorphenyl-cyanamid (100 mg, hergestellt, wie in J. Med. Chem., 1975, 18, 90-99 beschrieben) und C-(2-Methyl-[1,3]dioxolan-2-yl)-methylamin (69 mg) wurden im Kolben vermischt und 0,5 Stunden auf 150°C erhitzt. Das erkaltete Reaktionsgemisch wurde mit Wasser und Dichlormethan versetzt. Nach Trennen der Phasen wurde zweimal mit Dichlormethan extrahiert. Die vereinigten organischen Phasen wurden über Magnesiumsulfat getrocknet, filtriert und eingeengt. Der Rückstand wurde mittels präparativer HPLC gereinigt. Die Produkt enthaltenden Fraktionen wurden vereinigt, das Acetonitril am Rotationsverdampfer abgezogen und gefriergetrocknet. Es wurden 29 mg des gewünschten Guanidins erhalten.
   MS-Cl+: 304,2; LCMS-Rₜ (B)= 1,51 min
d) (2,6-Dichlorphenyl)-(4-methyl-1 H-imidazol-2-yl)-amin-Hydrochlorid N-(2,6-Dichlorphenyl)-N'-(2-methyl-[1,3]dioxolan-2-ylmethyl)-guanidin-Trifluoressigsäuresalz (29 mg) wurde mit 1 ml konzentrierter Salzsäure versetzt und 30 min. auf 90°C erhitzt. Nach Abkühlen wurde mit Wasser verdünnt und mit Dichlormethan versetzt. Dann wurde mit gesättigter Natriumhydrogencarbonat-Lösung alkalisch gestellt und zweimal mit Dichlormethan extrahiert. Die vereinigten organischen Phasen wurden über Magnesiumsulfat getrocknet, filtriert und eingeengt. Der Rückstand wurde mit Salzsäure versetzt und gefriergetrocknet. Es wurden 14 mg der Titelverbindung erhalten.
   MS-Cl+: 242,2; LCMS-Rₜ (B)= 1,42 min

### Beispiel 3: nicht erfindungsgemäß (2,6-Dichlorphenyl)-(4,5-dimethyl-1H-imidazol-2-yl)-amin-Hydrochlorid

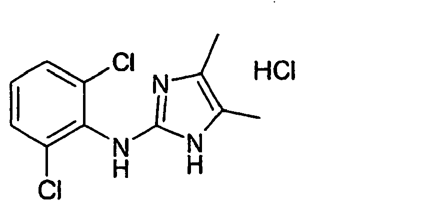

Ausgehend von 3-Brom-2-butanon wurde die Titelverbindung analog zu Beispiel 2 hergestellt.
MS-Cl+: 256,2; LCMS-Rₜ (C)= 1,43 min

### Beispiel 4: nicht erfindungsgemäß 2,6-Dichlorphenyl-(4-methyl-5-phenyl-1H-imidazol-2-yl)-amin-Hydrochlorid

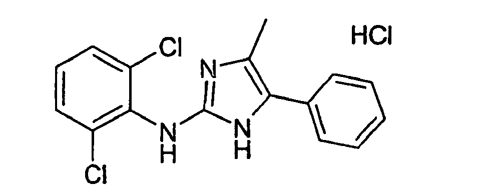

1-Amino-1-phenylaceton-Hydrochlorid (189 mg), 2,6-Dichlorphenyl-cyanamid (187 mg, hergestellt, wie in J. Med. Chem., 1975, 18, 90-99 beschrieben) und Triethylamin (101 mg) wurden in Ethanol (10 ml) 3 Stunden auf Rückflusstemperatur gehalten. Nach Abkühlen wurde im Vakuum zur Trockne gebracht und über präparative HPLC gereinigt. Die Produkt enthaltenden Fraktionen wurden vereinigt, das Acetonitril am Rotationsverdampfer abgezogen, der wässrige Rückstand mit Kaliumcarbonat neutralisiert und dreimal mit Essigester extrahiert. Nach Trocknen über Magnesiumsulfat wurde zur Trockne gebracht und nach Zugabe von Salzsäure gefriergetrocknet. Es wurden 26 mg der Titelverbindung erhalten.
MS-Cl+: 318,2; LCMS-Rₜ (A)= 4,14 min

### Beispiel 5: nicht erfindungsgemäß (2,6-Dichlorphenyl)-(4,5,6,7-tetrahydro-1H-benzoimidazol-2-yl)-amin-Hydrochlorid

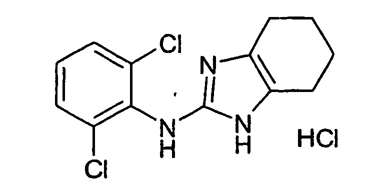

a) 1,4-Dioxa-spiro[4.5]dec-6-ylamin 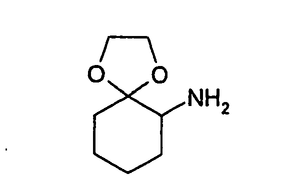 2-Chlorcyclohexanon (5 g) wurde in DMSO (10 ml) gelöst und Natriumazid (7,06 g) in DMSO (150 ml) gelöst langsam bei Raumtemperatur zugetropft. Anschließend wurde noch 60 Minuten bei Raumtemperatur gerührt. In das Reaktionsgemisch wurde Eiswasser gegeben und dann drei Mal mit n-Pentan (100 ml) extrahiert. Die vereinigten organischen Phasen wurden ein Mal mit Wasser gewaschen, über Magnesiumsulfat getrocknet und das Lösungsmittel nicht ganz zur Trockne eingeengt. Anschließend wurde mit Toluol koevaporiert, um restliches Pentan zu entfernen. Der Rückstand wurde mit abs. Toluol (120 ml) verdünnt, mit Ethylenglykol (4,8 g) und einer katalytischen Menge p-Toluolsulfonsäure versetzt und 5 Stunden am Wasserabscheider erhitzt. Nach Stehen über Nacht wurde weitere 6 Stunden am Wasserabscheider erhitzt. Außerdem wurden eine weitere Spatelspitze p-Toluolsulfonsäure und Ethylenglycol (6 ml) zugegeben. Anschließend wurde das Reaktionsgemisch mit Wasser gewaschen, und die organische Phase wurde noch einmal mit Toluol extrahiert. Die vereinigten organischen Phasen wurden über Magnesiumsulfat getrocknet und weitestgehend vom Lösungsmittel befreit. Der Rückstand wurde mit Methanol (40 ml) aufgenommen, in eine Schüttelapparatur überführt, mit Platindioxid (15 mg) versetzt und nach Zufuhr von Wasserstoff 2 Stunden hydriert. Das Platindioxid wurde abfiltriert und das Lösungsmittel eingeengt. Das Rohprodukt wurde über eine Kieselgelsäule chromatographiert (Laufmittel Dichlormethan/Methanol/konz. Ammoniak: 10:0,5:0,1). Es wurden 1,105 g eines öligen Produkts erhalten.
   MS-ES+: 158,2; LCMS-Rₜ (E)= 0,38 min
b) N-(2,6-Dichlorphenyl)-N'-(1,4-dioxa-spiro[4.5]dec-6-yl)-guanidin 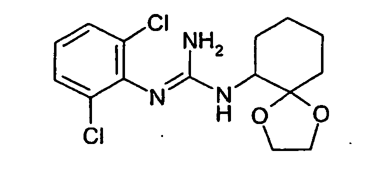 1,4-Dioxa-spiro[4.5]dec-6-ylamin (500 mg) wurde mit 2,6-Dichlorphenyl-cyanamid (595 mg, hergestellt, wie in J. Med. Chem., 1975, 18, 90-99 beschrieben) bei 130 °C zur Reaktion gebracht. Nach 45 Minuten wurde die Reaktion abgebrochen und das Reaktionsprodukt über präparative HPLC gereinigt. Die Produkt enthaltenden Fraktionen wurden vereinigt, das Acetonitril am Rotationsverdampfer abgezogen, der wässrige Rückstand mit Kaliumcarbonat neutralisiert und dreimal mit Dichlormethan extrahiert. Nach Trocknen über Magnesiumsulfat wurde zur Trockne gebracht. Es wurden 476 mg des gewünschten Guanidins erhalten. MS-ES+: 344,2; LCMS-Rₜ (D)= 2,10 min
c) (2,6-Dichlorphenyl)-(4,5,6,7-tetrahydro-1H-benzoimidazol-2-yl)-amin-Hydrochlorid
   N-(2,6-Dichlorphenyl)-N'-(1,4-dioxa-spiro[4.5]dec-6-yl)-guanidin (426 mg) wurde mit konzentrierter HCl (5 ml) versetzt und 5 Minuten bei Raumtemperatur gerührt. Der entstandene Niederschlag wurde abfiltriert und mit konzentrierter Salzsäure gewaschen. Bei der Verdünnung des Filtrats mit Wasser fiel erneut ein Niederschlag aus, der ebenfalls abfiltriert wurde. Die vereinigten Niederschläge ergaben 311 mg der gewünschten Verbindung.
   MS-ES+: 282,19; LCMS-Rₜ (D)= 2,01 min

### Beispiel 6 : 2-(2,6-Dichlorphenylamino)-1H-imidazol-4,5-dicarbonitril

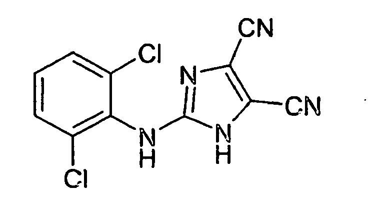

Diamino-maleinsäuredinitril (500 mg) wurde in absolutem Tetrahydrofuran (7,5 ml) gelöst und mit 2,6-Dichlorphenylisothiocyanat versetzt. Nach 3 Stunden Rühren bei Raumtemperatur und Stehenlassen über Nacht wurde das Lösungsmittel abgezogen und der Rückstand gereinigt. Die Produkt enthaltenden Fraktionen wurden vereinigt, das Acetonitril am Rotationsverdampfer abgezogen, der wässrige Rückstand mit Kaliumcarbonat neutralisiert und dreimal mit Essigester extrahiert. Nach Trocknen über Magnesiumsulfat wurde zur Trockne gebracht. Es wurden 73 mg des gewünschten Thioharnstoff-Intermediats erhalten. Dieses Produkt wurde mit N,N'-Dicyclohexylcarbodiimid (48 mg) in absolutem Tetrahydrofuran 5 Tage bei Raumtemperatur gerührt. Das Lösungsmittel wurde abgezogen und der Rückstand über präparative HPLC gereinigt. Die Produkt enthaltenden Fraktionen wurden vereinigt, das Acetonitril am Rotationsverdampfer abgezogen, der wässrige Rückstand mit Kaliumcarbonat neutralisiert und dreimal mit Essigester extrahiert. Nach Trocknen über Magnesiumsulfat wurde zur Trockne gebracht und der Rückstand gefriergetrocknet. Es wurden 16 mg des gewünschten Imidazols erhalten.
MS-Cl+: 278,2; LCMS-Rₜ (B)= 2,14 min

### Beispiele 7: (2,6-Dichlorphenyl)-methyl-(4,5,6,7-tetrahydro-1H-benzoimidazol-2-yl)-amin-Hydrochlorid

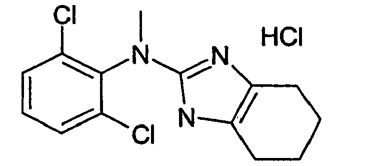

a) (2,6-Dichlorphenyl)-methyl-cyanamid 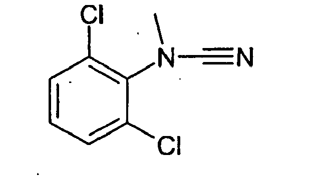 2,6-Dichlorphenyl-cyanamid (1 g, hergestellt, wie in J. Med. Chem., 1975, 18, 90-99 beschrieben) wurde in trockenem Dimethylformamid (25 ml) gelöst und mit gepulvertem Kaliumcarbonat (739 mg) versetzt. Nach fünf Minuten Rühren bei Raumtemperatur wurde Methyliodid (1,52 g) zugetropft und zwei Stunden bei Raumtemperatur gerührt. Nach Abziehen des Lösungsmittels wurde der Rückstand mit Wasser aufgenommen und drei Mal mit Ether extrahiert. Die vereinigten organischen Phasen wurde über Magnesiumsulfat getrocknet und filtriert. Nach Abziehen des Lösungsmittels wurde der Rückstand mittels präparativer HPLC gereinigt. Die Produkt enthaltenden Fraktionen wurden vereinigt, das Acetonitril am Rotationsverdampfer abgezogen, der wässrige Rückstand mit Kaliumcarbonat neutralisiert und drei Mal mit Essigester extrahiert. Nach Trocknen über Magnesiumsulfat wurde zur Trockne gebracht. Es wurden 600 mg des gewünschten Produkts erhalten. MS-ES+:201,1; LCMS-Rₜ (B)= 2,23 min
b) N-(2,6-Dichlorphenyl)-N'-(1,4-dioxa-spiro[4.5]dec-6-yl)-N-methyl-guanidin 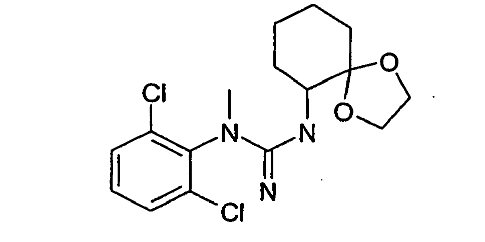 Zu einer siedenden Lösung aus 1,4-Dioxa-spiro[4.5]dec-6-ylamin (195 mg, Beispiel 5) in trockenem THF (5 ml) wurde eine Lösung aus (2,6-Dichlorphenyl)-methyl-cyanamid (25 mg) gelöst in trockenem Tetrahydrofuran (5 ml) getropft und das Tetrahydrofuran im Vakuum entfernt. Der Rückstand wurde dann 15 Minuten im Ölbad bei 130 °C erhitzt und anschließend mittels präparativer HPLC gereinigt. Die Produkt enthaltenden Fraktionen wurden vereinigt, das Acetonitril am Rotationsverdampfer abgezogen, der wässrige Rückstand mit Kaliumcarbonat neutralisiert und dreimal mit Essigester extrahiert. Nach Trocknen über Magnesiumsulfat wurde zur Trockne gebracht und der Rückstand gefriergetrocknet. Es wurden 18 mg des gewünschten Produkts erhalten.
   MS-ES+: 358,4; LCMS-Rₜ (E)= 1,48 min
c) (2,6-Dichlorphenyl)-methyl-(4,5,6,7-tetrahydro-1H-benzoimidazol-2-yl)-amin-Hydrochlorid
   Zu N-(2,6-Dichlorphenyl)-N'-(1,4-dioxa-spiro[4.5]dec-6-yl)-N-methyl-guanidin (18 mg) wurde konzentrierte HCl (1 ml) gegeben. Nach 10 Minuten wurde mit Wasser verdünnt und gefriergetrocknet. Das erhaltene Produkt wurde drei Mal mit Toluol koevaporiert. Es wurden 16 mg der Titelverbindung erhalten.
   MS-ES+: 296,17; LCMS-Rₜ (D)= 2,12 min

### Pharmakologische Daten:

### Testbeschreibung:

In diesem Test wurde die Erholung des intrazellulären pHs (pHᵢ) nach einer Ansäuerung ermittelt, die bei funktionsfähigem NHE3 auch unter bicarbonatfreien Bedingungen einsetzt. Dazu wurde der pHᵢ mit dem pH-sensitiven Fluoreszenzfarbstoff BCECF (Calbiochem, eingesetzt wird die Vorstufe BCECF-AM) bestimmt. Die Zellen (Fibroblasten, LAP1 Zellen) wurden zunächst mit BCECF beladen. Die BCECF-Fluoreszenz wurde in einem "Ratio Fluorescence Spectrometer" (Photon Technology International, South Brunswick, N.J., USA) bei Anregungswellenlängen von 505 und 440 nm und einer Emissionswellenlänge von 535 nm bestimmt und mittels Kalibrierungskurven in den pHᵢ umgerechnet. Die Zellen wurden bereits bei der BCECF-Beladung in NH₄Cl-Puffer (pH 7,4) inkubiert (NH₄Cl-Puffer: 115 mM NaCl, 20 mM NH₄Cl, 5 mM KCl, 1 mM CaCl₂, 1 mM MgSO₄, 20 mM Hepes, 5 mM Glucose, 1 mg/ml BSA; mit 1 M NaOH wird ein pH von 7,4 eingestellt). Die intrazelluläre Ansäuerung wurde durch Zugabe von 975 µl eines NH₄Cl -freien Puffers (s. u.) zu 25 µl Aliquots der in NH₄Cl -Puffer inkubierten Zellen induziert. Die nachfolgende Geschwindigkeit der pH-Erholung wurde drei Minuten registriert. Für die Berechnung der inhibitorischen Potenz der getesteten Substanzen wurden die Zellen zunächst in Puffern untersucht, bei denen eine vollständige bzw. überhaupt keine pH-Erholung stattfand. Zur vollständigen pH-Erholung (100%) wurden die Zellen in Na⁺-haltigem Puffer inkubiert (133,8 mM NaCl, 4,7 mM KCl, 1,25 mM CaCl₂, 1,25 mM MgCl₂, 0,97 mM Na₂HPO₄, 0,23 mM NaH₂PO₄, 5 mM Hepes, 5 mM Glucose, mit 1 M NaOH wird ein pH von 7,0 eingestellt). Für die Bestimmung des 0%-Wertes wurden die Zellen in einem Na⁺-freien Puffer inkubiert (133,8 mM Cholinchlorid, 4,7 mM KCl, 1,25 mM CaCl₂, 1,25 mM MgCl₂, 0.97 mM K₂HPO₄, 0,23 mM KH₂PO₄, 5 mM Hepes, 5 mM Glucose, mit 1 M KOH wird ein pH von 7,0 eingestellt). Die zu testenden Substanzen wurden in dem Na⁺-haltigem Puffer angesetzt. Die Erholung des intrazellulären pH's bei jeder getesteten Konzentration einer Substanz wurde in Prozent der maximalen Erholung ausgedrückt. Aus den Prozentwerten der pH-Erholung wurde mittels des Programms Sigma-Plot der IC₅₀-Wert der jeweiligen Substanz für die einzelnen NHE-Subtypen berechnet.

### Ergebnisse:

| Beispiel | IC₅₀ [µM], (rNHE3) |
|---|---|
| Clonidin | 620*) |
| 1 ** | 7,7 |
| 2 ** | 1,7 |
| 3 ** | 0,84 |
| 5 | 0,24 |
| 6** | 98 |
| 7 | 0,28 |

| | |
|---|---|
| *) Lit.: J. Orlowski et al, J. Biol. Chem. 268, 25536 ** nicht erfindungsgemäß | |

## Patentansprüche

1. Verbindungen der Formel I, 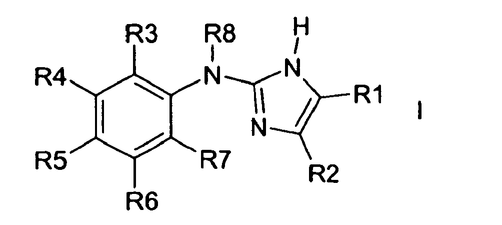 worin bedeuten:
R1 und R2 zusammen mit den beiden Kohlenstoffatomen, an die sie gebunden sind, einen fünf-, sechs-, sieben- oder achtgliedrigen, eine oder zwei Doppelbindungen enthaltenden Kohlenstoffring,
wobei der Ring unsubstituiert ist oder mit 1, 2, 3, 4, 5, 6, 7, 8, 9, 10, 11 oder 12 F-Atomen und/oder mit einem oder zwei Resten aus der Gruppe OH, NR9R10, Alkyl mit 1, 2, 3 oder 4 C-Atomen, CN, CF₃ oder Alkoxy mit 1, 2, 3 oder 4 C-Atomen substituiert ist;
R9 und R10 unabhängig voneinander H oder Alkyl mit 1, 2, 3 oder 4 C-Atomen;
R3, R4, R5, R6 und R7 unabhängig voneinander H, F, Cl, Br, I, Alkyl mit 1, 2, 3 oder 4 C-Atomen, oder Alkoxy mit 1, 2, 3 oder 4 C-Atomen,
wobei die Kohlenstoffketten unsubstituiert oder unabhängig voneinander mit 1, 2, 3, 4, 5, 6, 7, 8 oder 9 F-Atomen substituiert sind,
wobei R3 und R7 nicht beide gleichzeitig Wasserstoff entsprechen;
R8 H, Alkyl mit 1, 2, 3 oder 4 C-Atomen oder Cycloalkyl mit 3, 4 oder 5 C-Atomen,
wobei die Kohlenstoffketten oder Cycloalkylreste unsubstituiert oder mit 1, 2, 3, 4, 5, 6, 7, 8 oder 9 F-Atomen substituiert sind:
sowie deren pharmazeutisch verträgliche Salze und Trifluoressigsäuresalze.

2. Verbindungen der Formel I nach Anspruch 1, worin bedeuten:
R1 und R2 zusammen mit den beiden Kohlenstoffatomen, an die sie gebunden sind, einen fünf-, sechs-, sieben- oder achtgliedrigen, eine oder zwei Doppelbindungen enthaltenden Kohlenstoffring,
wobei der Ring unsubstituiert oder mit 1, 2, 3, 4, 5, 6, 7, 8, 9, 10, 11 oder 12 F-Atomen und/oder mit ein oder zwei Resten aus der Gruppe CH₃ oder OCH₃ substituiert ist;
R3, R4, R5, R6 und R7 unabhängig voneinander H, F, Cl, Br, Alkyl mit 1, 2, 3 oder 4 C-Atomen oder Alkoxy mit 1, 2, 3 oder 4 C-Atomen,
wobei die Kohlenstoffketten unsubstituiert oder unabhängig voneinander mit 1, 2, 3, 4, 5, 6, 7, 8 oder 9 F-Atomen substituiert sind,
wobei R3 und R7 nicht beide gleichzeitig Wasserstoff entsprechen;
R8 H, Alkyl mit 1, 2, 3 oder 4 C-Atomen oder Cycloalkyl mit 3, 4 oder 5 C-Atomen,
wobei die Kohlenstoffketten oder Cycloalkylreste unsubstituiert oder mit 1, 2, 3, 4, 5, 6, 7, 8 oder 9 F-Atomen substituiert sind;
sowie deren pharmazeutisch verträgliche Salze und Trifluoressigsäuresalze.

3. Verbindungen der Formel I nach Anspruch 2, worin bedeuten:
R1 und R2 zusammen mit den beiden Kohlenstoffatomen, an die sie gebunden sind, einen fünf- , sechs- oder siebengliedrigen, eine Doppelbindung enthaltenden Kohlenstoffring,
R3 und R7 unabhängig voneinander F, Cl, Br oder Alkyl mit 1, 2, 3 oder 4 C-Atomen,
wobei die Kohlenstoffketten unsubstituiert oder unabhängig voneinander mit 1, 2, 3, 4, 5, 6, 7, 8 oder 9 F-Atomen substituiert sind;
R4, R5 und R6 H
R8 H oder Alkyl mit 1, 2, 3 oder 4 C-Atomen, wobei die Kohlenstoffketten unsubstituiert oder mit 1, 2, 3, 4, 5, 6, 7, 8 oder 9 F-Atomen substituiert sind; sowie deren pharmazeutisch verträgliche Salze und Trifluoressigsäuresalze.

4. Verbindungen nach Anspruch 3, **dadurch gekennzeichnet, dass** sie ausgewählt sind aus der Gruppe:
(2,6-Dichlorphenyl)-(4,5,6,7-tetrahydro-1 H-benzoimidazol-2-yl)-amin- oder
(2,6-Dichlorphenyl)-methyl-(4,5,6,7-tetrahydro-1H-benzoimidazol-2-yl)-amin
sowie deren pharmazeutisch verträgliche Salze und Trifluoressigsäuresalze.

5. Verbindungen der Formel l und deren pharmazeutisch verträgliche Salze nach einem oder mehreren der Ansprüche 1 bis 4 zur Verwendung als Medikament.

6. Verwendung einer Verbindungen der Formel I und/oder deren pharmazeutisch verträgliche Salze nach einem oder mehreren der Ansprüche 1 bis 4 zur Herstellung eines Medikaments zur Behandlung oder Prophylaxe von Störungen des Atemantriebs, von Atemstörungen, Schlaf-bedingten Atemstörungen, Schlafapnoen, von Schnarchen, von akuten und chronischen Nierenerkrankungen, des akuten Nierenversagens und des chronischen Nierenversagens, von Störungen der Darmfunktion, des Bluthochdrucks, der essentiellen Hypertonie, von Erkrankungen des Zentralnervensystems, von Erkrankungen; die durch ZNS-Übererregbarkeit resultieren, Epilepsie und zentral ausgelöste Krämpfe oder von Angstzuständen, Depressionen und Psychosen, von ischämischen Zuständen des peripheren oder zentralen Nervensystems oder des Schlaganfalls, von akuten und chronischen Schäden und Erkrankungen peripherer Organe oder Gliedmaßen, die durch lschämie- oder durch Reperfusionsereignisse verursacht werden, von Atherosklerose, von Störungen des Fettstoffwechsels, von Thrombosen, von Störungen der Gallenfunktion, von Befall durch Ektoparasiten, von Erkrankungen infolge endothelialer Dysfunktion, von Protozoen-Erkrankungen, der Malaria, zur Konservierung und Lagerung von Transplantaten für chirurgische Maßnahmen, für den Einsatz bei chirurgischen Operationen und Organtransplantationen oder zur Behandlung von Schockzuständen oder der Zuckerkrankheit und diabetischer Spätschäden oder von Krankheiten, bei denen die Zellproliferation eine primäre oder sekundäre Ursache darstellt und zur Gesunderhaltung und Lebensverlängerung.

7. Verwendung einer Verbindung der Formel I und/oder deren pharmazeutisch verträgliche Salze nach einem oder mehreren der Ansprüche der Ansprüche 1 bis 4 in Kombination mit anderen Arzneimitteln oder Wirkstoffen zur Herstellung eines Medikaments zur Behandlung oder Prophylaxe von Störungen des Atemantriebs, von Atemstörungen, Schlaf-bedingten Atemstörungen, Schlafapnoen, von Schnarchen, von akuten und chronischen Nierenerkrankungen, des akuten Nierenversagens und des chronischen Nierenversagens, von Störungen der Darmfunktion, des Bluthochdrucks, der essentiellen Hypertonie, von Erkrankungen des Zentralnervensystems, von Erkrankungen, die durch ZNS-Übererregbarkeit resultieren, Epilepsie und zentral ausgelöste Krämpfe oder von Angstzuständen, Depressionen und Psychosen, von ischämischen Zuständen des peripheren oder zentralen Nervensystems oder des Schlaganfalls, von akuten und chronischen Schäden und Erkrankungen peripherer Organe oder Gliedmaßen, die durch Ischämie- oder durch Reperfusionsereignisse verursacht werden, von Atherosklerose, von Störungen des Fettstoffwechsels, von Thrombosen, von Störungen der Gallenfunktion, von Befall durch Ektoparasiten, von Erkrankungen infolge endothelialer Dysfunktion, von Protozoen-Erkrankungen, der Malaria, zur Konservierung und Lagerung von Transplantaten für chirurgische Maßnahmen, für den Einsatz bei chirurgischen Operationen und Organtransplantationen oder zur Behandlung von Schockzuständen oder der Zuckerkrankheit und diabetischer Spätschäden oder von Krankheiten, bei denen die Zellproliferation eine primäre oder sekundäre Ursache darstellt und zur Gesunderhaltung und Lebensverlängerung.

8. Verwendung einer Verbindung der Formel I und/oder deren pharmazeutisch verträgliche Salze nach einem oder mehreren der Ansprüche 1 bis 4 allein oder in Kombination mit anderen Arzneimitteln oder Wirkstoffen zur Herstellung eines Medikaments zur Behandlung oder Prophylaxe von Störungen des Atemantriebs und/oder von Schlaf-bedingten Atemstörungen und Schlafapnoen.

9. Verwendung einer Verbindung der Formel I und/oder deren pharmazeutisch verträgliche Salze nach einem oder mehreren der Ansprüche 1 bis 4 allein oder in Kombination mit anderen Arzneimitteln oder Wirkstoffen zur Herstellung eines Medikaments zur Behandlung oder Prophylaxe des Schnarchens.

10. Verwendung einer Verbindung der Formel I und/oder deren pharmazeutisch verträgliche Salze nach einem oder mehreren der Ansprüche 1 bis 4 allein oder in Kombination mit anderen Arzneimitteln oder Wirkstoffen zur Herstellung eines Medikaments zur Behandlung oder zur Prophylaxe von akuten und chronischen Nierenerkrankungen, des akuten Nierenversagens oder des chronischen Nierenversagens.

11. Verwendung einer Verbindung der Formel I und/oder deren pharmazeutisch verträgliche Salze nach einem oder mehreren der Ansprüche 1 bis 4 allein oder in Kombination mit anderen Arzneimitteln oder Wirkstoffen zur Herstellung eines Medikaments zur Behandlung oder Prophylaxe von Störungen der Darmfunktion.

12. Heilmittel für die humane, veterinäre oder phytoprotektive Anwendung enthaltend eine wirksame Menge einer oder mehreren Verbindungen der Formel I und/oder deren pharmazeutisch verträgliche Salze nach einem oder mehreren der Ansprüche 1 bis 4.

13. Heilmittel für die humane, veterinäre oder phytoprotektive Anwendung enthaltend eine wirksame Menge einer oder mehrerer Verbindungen der Formel I und/oder deren pharmazeutisch verträgliche Salze nach einem oder mehreren der Ansprüche 1 bis 4 in Kombination mit einem oder mehreren anderen pharmakologischen Wirkstoffen oder Arzneimitteln.

14. Verwendung von Verbindungen der Formel I 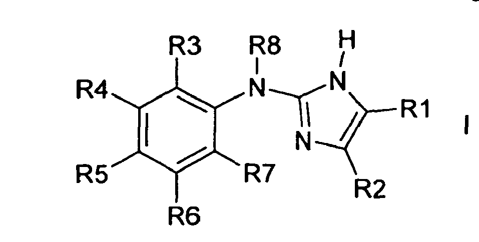 und/oder von deren pharmazeutisch verträglichen Salzen zur Herstellung eines Medikamentes zur Behandlung von Krankheiten, welche durch NHE-Inhibitoren beeinflusst werden können, worin bedeuten:
R1 und R2 zusammen mit den beiden Kohlenstoffatomen, an die sie gebunden sind, einen fünf-, sechs-, sieben- oder achtgliedrigen, eine oder zwei Doppelbindungen enthaltenden Kohlenstoffring,
wobei der Ring unsubstituiert ist oder mit 1, 2, 3, 4, 5, 6, 7, 8, 9, 10, 11 oder 12 F-Atomen und/oder ein oder zwei Resten aus der Gruppe NR9R10, Alkyl mit 1, 2, 3 oder 4 C-Atomen, OH, CN, CF₃ oder Alkoxy mit 1, 2, 3 oder 4 C-Atomen substituiert ist mit
R9, R10 unabhängig voneinander H, Alkyl mit 1, 2, 3 oder 4 C-Atomen;
R3, R4, R5, R6 oder R7 unabhängig voneinander H, F, Cl, Br, l, Alkyl mit 1, 2, 3 oder 4 C-Atomen, (C₂-C₄)-Alkenyl, Cycloalkyl mit 3, 4, 5 oder 6 C-Atomen, OH, Alkoxy mit 1, 2, 3 oder 4 C-Atomen, CN, NO₂, NH₂, Alkylamino mit 1, 2, 3 oder 4 C-Atomen oder Dialkylamino mit jeweils 1, 2, 3 oder 4 C-Atomen,
wobei die Kohlenstoffketten oder -ringe unsubstituiert oder unabhängig voneinander mit 1, 2, 3, 4, 5, 6, 7, 8 oder 9 F-Atomen substituiert sind;
wobei R3 und R7 nicht gleichzeitig Wasserstoff entsprechen;
R8 H, Alkyl mit 1, 2, 3 oder 4 C-Atomen oder Cycloalkyl mit 3, 4 oder 5 C-Atomen, wobei die Kohlenstoffketten oder -ringe unsubstituiert oder mit 1, 2, 3, 4, 5, 6, 7, 8 oder 9 F-Atomen substituiert sind.

15. Verwendung einer Verbindung der Formel I und/oder deren pharmazeutisch verträgliche Salze nach Anspruch 14 zur Herstellung eines Medikaments zur Behandlung oder Prophylaxe von Störungen des Atemantriebs, von Atemstörungen und Schlaf-bedingten Atemstörungen wie Schlafapnoen, des Schnarchens, von akuten und chronischen Nierenerkrankungen, des akuten Nierenversagens und des chronischen Nierenversagens, von Störungen der Darmfunktion, von Erkrankungen des Zentralnervensystems, von Erkrankungen, die durch ZNS-Übererregbarkeit resultieren, Epilepsie und zentral ausgelöste Krämpfe, von Angstzuständen, Depressionen und Psychosen, von ischämischen Zuständen des peripheren und zentralen Nervensystems und des Schlaganfalls, von akuten und chronischen Schäden und Erkrankungen peripherer Organe und Gliedmaßen, die durch lschämie- oder durch Reperfusionsereignisse verursacht werden, zur Konservierung und Lagerung von Transplantaten für chirurgische Maßnahmen, für den Einsatz bei chirurgischen Operationen und Organtransplantationen, zur Behandlung von Schockzuständen, zur Behandlung der Zuckerkrankheit und diabetischer Spätschäden, der Atherosklerose, von Störungen des Fettstoffwechsels, von Krankheiten, bei denen die Zellproliferation eine primäre oder sekundäre Ursache darstellt, von Thrombosen, von Störungen der Gallenfunktion, zur Behandlung oder Prophylaxe des Befalls durch Ektoparasiten, zur Behandlung oder Prophylaxe von Erkrankungen infolge endothelialer Dysfunktion, zur Gesunderhaltung und Lebensverlängerung und zur Behandlung oder Prophylaxe von Protozoen-Erkrankungen und der Malaria.

16. Verfahren zur Herstellung von α-Aminoketalen der Formel V 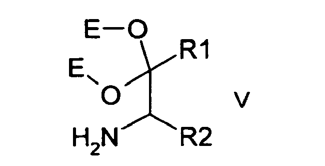 worin bedeuten:
R1 und R2 zusammen mit den beiden Kohlenstoffatomen, an die sie gebunden sind, einen fünf- bis achtgliedrigen gesättigten oder eine Doppelbindung enthaltenden Kohlenstoffring,
wobei der Ring unsubstituiert oder mit 1, 2, 3, 4, 5, 6, 7, 8, 9, 10, 11 oder 12 F-Atomen und/oder mit ein oder zwei Resten aus der Gruppe NR11R12, Alkyl mit 1, 2, 3 oder 4 C-Atomen, CN, CF₃ oder Alkoxy mit 1, 2, 3 oder 4 C-Atomen substituiert ist mit
R11, R12 unabhängig voneinander Alkyl mit 1, 2, 3 oder 4 C-Atomen, Benzyl, 4-Methoxybenzyl;
E Alkyl mit 1, 2, 3 oder 4 C-Atomen oder beide Reste E ein cyclisches Ketal ausbilden, in dem E-E (C₂- C₄)-Alkylen ist;
Hal Cl, Br, I
**dadurch gekennzeichnet,**
**dass** ein α-Halogenketon oder -aldehyd der Formel VI mit einem Azid unter Azid-Halogen-Austausch in das entsprechende α-Azidketon oder -aldehyd der Formel VII überführt wird, 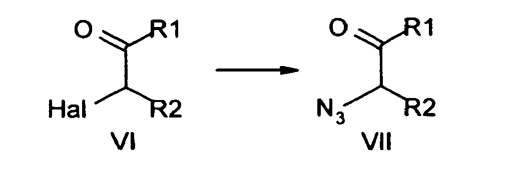 das anschließend mit ein- oder zweiwertigen Alkoholen unter Ketalisierung oder Acetalisierung zum α-Azidketal oder -acetal der Formel VIII umgesetzt wird, 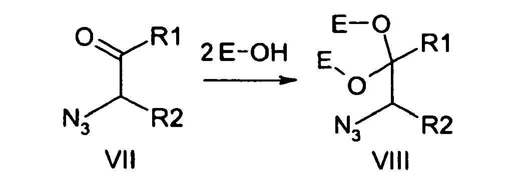 welches dann zu Verbindungen der Formel V reduziert wird.

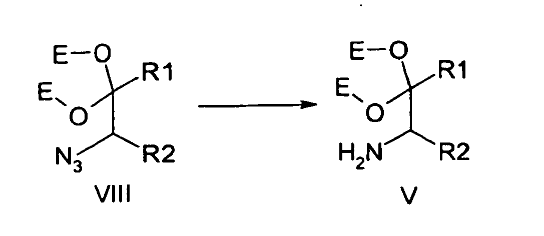

## Claims

1. A compound of the formula I 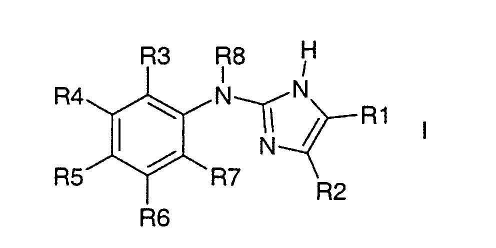 in which the meanings are:
R1 and R2 together with the two carbon atoms to which they are bonded a five-, six-, seven- or eight-membered carbon ring comprising one or two double bonds,
where the ring is unsubstituted or substituted by 1, 2, 3, 4, 5, 6, 7, 8, 9, 10, 11 or 12 F atoms and/or by one or two radicals from the group of OH, NR9R10, alkyl with 1, 2, 3 or 4 carbon atoms, CN, CF₃ or alkoxy with 1, 2, 3 or 4 carbon atoms;
R9 and R10 independently of one another H or alkyl with 1, 2, 3 or 4 carbon atoms;
R3, R4, R5, R6 and R7 independently of one another H, F, Cl, Br, I, alkyl with 1, 2, 3 or 4 carbon atoms, or alkoxy with 1, 2, 3 or 4 carbon atoms, where the carbon chains are unsubstituted or substituted independently of one another by 1, 2, 3, 4, 5, 6, 7, 8 or 9 F atoms,
where R3 and R7 do not both correspond simultaneously to hydrogen;
R8 H, alkyl with 1, 2, 3 or 4 carbon atoms or cycloalkyl with 3, 4 or 5 carbon atoms,
where the carbon chains or cycloalkyl radicals are unsubstituted or substituted by 1, 2, 3, 4, 5, 6, 7, 8 or 9 F atoms;
and the pharmaceutically acceptable salts and trifluoroacetic acid salts thereof.

2. A compound of the formula I as claimed in claim 1, in which the meanings are:
R1 and R2 together with the two carbon atoms to which they are bonded a five-, six-, seven- or eight-membered carbon ring comprising one or two double bonds,
where the ring is unsubstituted or substituted by 1, 2, 3, 4, 5, 6, 7, 8, 9, 10, 11 or 12 F atoms and/or by one or two radicals from the group of CH₃ or OCH₃;
R3, R4, R5, R6 and R7 independently of one another H, F, Cl, Br, alkyl with 1, 2, 3 or 4 carbon atoms or alkoxy with 1, 2, 3 or 4 carbon atoms,
where the carbon chains are unsubstituted or substituted independently of one another by 1, 2, 3, 4, 5, 6, 7, 8 or 9 F atoms,
where R3 and R7 do not both correspond simultaneously to hydrogen;
R8 H, alkyl with 1, 2, 3 or 4 carbon atoms or cycloalkyl with 3, 4 or 5 carbon atoms,
where the carbon chains or cycloalkyl radicals are unsubstituted or substituted by 1, 2, 3, 4, 5, 6, 7, 8 or 9 F atoms;
and the pharmaceutically acceptable salts and trifluoroacetic acid salts thereof.

3. A compound of the formula I as claimed in claim 2, in which the meanings are:
R1 and R2 together with the two carbon atoms to which they are bonded a five-, six- or seven-membered carbon ring comprising a double bond,
R3 and R7 independently of one another F, Cl, Br or alkyl with 1, 2, 3 or 4 carbon atoms,
where the carbon chains are unsubstituted or substituted independently of one another by 1, 2, 3, 4, 5, 6, 7, 8 or 9 F atoms;
R4, R5 and R6 H
R8 H or alkyl with 1, 2, 3 or 4 carbon atoms,
where the carbon chains are unsubstituted or substituted by 1, 2, 3, 4, 5, 6, 7, 8 or 9 F atoms;
and the pharmaceutically acceptable salts and trifluoroacetic acid salts thereof.

4. A compound as claimed in claim 3, which is selected from the group: (2,6-dichlorophenyl)(4,5,6,7-tetrahydro-1 H-benzoimidazol-2-yl)amine or (2,6-dichlorophenyl)methyl(4,5,6,7-tetrahydro-1H-benzoimidazol-2-yl)amine and the pharmaceutically acceptable salts and trifluoroacetic acid salts thereof.

5. A compound of the formula I and the pharmaceutically acceptable salts thereof as claimed in one or more of claims 1 to 4 for use as medicament.

6. The use of compounds of the formula I and/or the pharmaceutically acceptable salts thereof as claimed in one or more of claims 1 to 4 for producing a medicament for the treatment or prophylaxis of disorders of respiratory drive, of respiratory disorders, sleep-related respiratory disorders, sleep apneas, of snoring, of acute and chronic renal disorders, of acute renal failure and of chronic renal failure, of disorders of intestinal function, of high blood pressure, of essential hypertension, of disorders of the central nervous system, of disorders which result from CNS overexcitability, epilepsy and centrally induced convulsions or of anxiety states, depressions and psychoses, of ischemic states of the peripheral or central nervous system or of stroke, of acute and chronic damage to and disorders of peripheral organs or limbs caused by ischemic or by reperfusion events, of atherosclerosis, of disorders of lipid metabolism, of thromboses, of disorders of biliary function, of infestation by ectoparasites, of disorders resulting from endothelial dysfunction, of protozoal disorders, of malaria, for preservation and storage of transplants for surgical procedures, for use in surgical operations and organ transplantations or for the treatment of states of shock or of diabetes and late damage from diabetes or of disorders in which cellular proliferation represents a primary or secondary cause, and for maintaining health and prolonging life.

7. The use of a compound of the formula I and/or the pharmaceutically acceptable salts thereof as claimed in one or more of claims 1 to 4 in combination with other drugs or active ingredients for producing a medicament for the treatment or prophylaxis of disorders of respiratory drive, of respiratory disorders, sleep-related respiratory disorders, sleep apneas, of snoring, of acute and chronic renal disorders, of acute renal failure and of chronic renal failure, of disorders of intestinal function, of high blood pressure, of essential hypertension, of disorders of the central nervous system, of disorders which result from CNS overexcitability, epilepsy and centrally induced convulsions or of anxiety states, depressions and psychoses, of ischemic states of the peripheral or central nervous system or of stroke, of acute and chronic damage to and disorders of peripheral organs or limbs caused by ischemic or by reperfusion events, of atherosclerosis, of disorders of lipid metabolism, of thromboses, of disorders of biliary function, of infestation by ectoparasites, of disorders resulting from endothelial dysfunction, of protozoal disorders, of malaria, for preservation and storage of transplants for surgical procedures, for use in surgical operations and organ transplantations or for the treatment of states of shock or of diabetes and late damage from diabetes or of disorders in which cellular proliferation represents a primary or secondary cause, and for maintaining health and prolonging life.

8. The use of a compound of the formula I and/or the pharmaceutically acceptable salts thereof as claimed in one or more of claims 1 to 4 alone or in combination with other drugs or active ingredients for producing a medicament for the treatment or prophylaxis of disorders of respiratory drive and/or of sleep-related respiratory disorders and sleep apneas.

9. The use of a compound of the formula I and/or the pharmaceutically acceptable salts thereof as claimed in one or more of claims 1 to 4 alone or in combination with other drugs or active ingredients for producing a medicament for the treatment or prophylaxis of snoring.

10. The use of a compound of the formula I and/or the pharmaceutically acceptable salts thereof as claimed in one or more of claims 1 to 4 alone or in combination with other drugs or active ingredients for producing a medicament for the treatment or prophylaxis of acute and chronic renal disorders, of acute renal failure or of chronic renal failure.

11. The use of a compound of the formula I and/or the pharmaceutically acceptable salts thereof as claimed in one or more of claims 1 to 4 alone or in combination with other drugs or active ingredients for producing a medicament for the treatment or prophylaxis of disorders of intestinal function.

12. A curative composition for human, veterinary or phytoprotective use comprising an effective amount of one or more compounds of the formula I and/or the pharmaceutically acceptable salts thereof as claimed in one or more of claims 1 to 4.

13. A curative composition for human, veterinary or phytoprotective use comprising an effective amount of one or more compounds of the formula I and/or the pharmaceutically acceptable salts thereof as claimed in one or more of claims 1 to 4, in combination with one or more other pharmacological active ingredients or drugs.

14. The use of compounds of the formula I 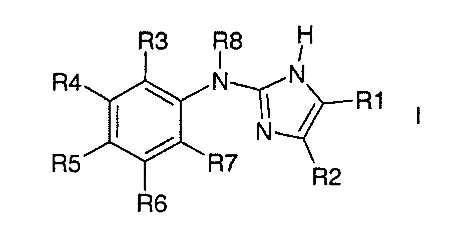 and/or of the pharmaceutically acceptable salts thereof for producing a medicament for the treatment of disorders which can be influenced by NHE inhibitors, in which the meanings are:
R1 and R2 together with the two carbon atoms to which they are bonded a five-, six-, seven- or eight-membered carbon ring comprising one or two double bonds,
where the ring is unsubstituted or substituted by 1, 2, 3, 4, 5, 6, 7, 8, 9, 10, 11 or 12 F atoms and/or one or two radicals from the group of NR9R10, alkyl with 1, 2, 3 or 4 carbon atoms, OH, CN, CF₃ or alkoxy with 1, 2, 3 or 4 carbon atoms, with
R9, R10 independently of one another H, alkyl with 1, 2, 3 or 4 carbon atoms;
R3, R4, R5, R6 or R7 independently of one another H, F, Cl, Br, I, alkyl with 1, 2, 3 or 4 carbon atoms, (C₂-C₄)-alkenyl, cycloalkyl with 3, 4, 5 or 6 carbon atoms, OH, alkoxy with 1, 2, 3 or 4 carbon atoms, CN, NO₂, NH₂, alkylamino with 1, 2, 3 or 4 carbon atoms or dialkylamino with, in each case, 1, 2, 3 or 4 carbon atoms,
where the carbon chains or rings are unsubstituted or substituted independently of one another by 1, 2, 3, 4, 5, 6, 7, 8 or 9 F atoms;
where R3 and R7 do not correspond simultaneously to hydrogen;
R8 H, alkyl with 1, 2, 3 or 4 carbon atoms or cycloalkyl with 3, 4 or 5 carbon atoms,
where the carbon chains or rings are unsubstituted or substituted by 1, 2, 3, 4, 5, 6, 7, 8 or 9 F atoms.

15. The use of a compound of the formula I and/or the pharmaceutically acceptable salts thereof as claimed in claim 14 for producing a medicament for the treatment or prophylaxis of disorders of respiratory drive, of respiratory disorders and sleep-related respiratory disorders such as sleep apneas, of snoring, of acute and chronic renal disorders, of acute renal failure and of chronic renal failure, of disorders of intestinal function, of disorders of the central nervous system, of disorders resulting from CNS overexcitability, epilepsy and centrally induced convulsions, of anxiety states, depressions and psychoses, of ischemic states of the peripheral and central nervous system and of stroke, of acute and chronic damage to and disorders of peripheral organs and limbs caused by ischemic or by reperfusion events, for the preservation and storage of transplants for surgical procedures, for use in surgical operations and organ transplantations, for the treatment of states of shock, for the treatment of diabetes and late damage from diabetes, of atherosclerosis, of disorders of lipid metabolism, of disorders in which cellular proliferation represents a primary or secondary cause, of thromboses, of disorders of biliary function, for the treatment or prophylaxis of infestation by ectoparasites, for the treatment or prophylaxis of disorders resulting from endothelial dysfunction, for maintaining health and prolonging life and for the treatment or prophylaxis of protozoal disorders, and of malaria.

16. A process for preparing α-amino ketals of the formula V 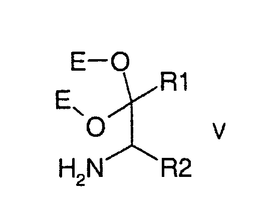 in which the meanings are:
R1 and R2 together with the two carbon atoms to which they are bonded a five- to eight-membered carbon ring which is saturated or comprises a double bond,
where the ring is unsubstituted or substituted by 1, 2, 3, 4, 5, 6, 7, 8, 9, 10, 11 or 12 F atoms and/or by one or two radicals from the group of NR11 R12, alkyl with 1, 2, 3 or 4 carbon atoms, CN, CF₃ or alkoxy with 1, 2, 3 or 4 carbon atoms, with
R11, R12 independently of one another alkyl with 1, 2, 3 or 4 carbon atoms, benzyl, 4-methoxybenzyl;
E alkyl with 1, 2, 3 or 4 carbon atoms, or the two E radicals form a cyclic ketal in which E-E is (C₂-C₄)-alkylene;
Hal Cl, Br, I
which comprises
converting an α-halo ketone or aldehyde of the formula VI with an azide by azide-halogen exchange into the corresponding α-azide ketone or aldehyde of the formula VII 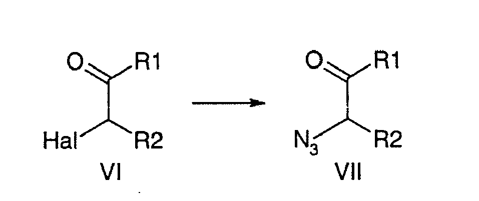 which is subsequently reacted with mono- or dihydric alcohols by ketalization or acetalization to give the α-azide ketal or acetal of the formula VIII 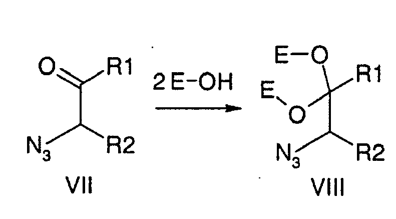 which is then reduced to compounds of the formula V.

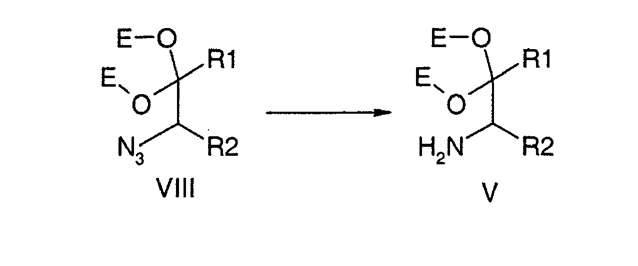

## Revendications

1. Composés de formule I, 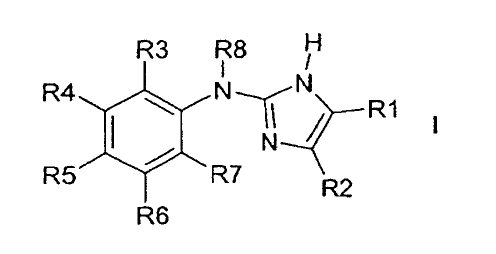 dans laquelle :
R1 et R2 forment ensemble, avec les deux atomes de carbone auxquels ils sont fixés, un cycle carboné à cinq, six, sept ou huit chaînons, comportant une ou deux doubles liaisons,
le cycle étant non substitué ou substitué par 1, 2, 3, 4, 5, 6, 7, 8, 9, 10, 11 ou 12 atomes de fluor et/ou par un ou deux radicaux choisis dans l'ensemble constitué par OH, NR9R10, alkyle ayant 1, 2, 3 ou 4 atomes de carbone, CN, CF₃ ou alcoxy ayant 1, 2, 3 ou 4 atomes de carbone ;
R9 et R10 représentent, indépendamment l'un de l'autre, H ou un groupe alkyle ayant 1, 2, 3 ou 4 atomes de carbone ;
R3, R4, R5, R6 et R7 représentent, indépendamment les uns des autres, H, F, Cl, Br, I, un groupe alkyle ayant 1, 2, 3 ou 4 atomes de carbone ou alcoxy ayant 1, 2, 3 ou 4 atomes de carbone,
les chaînes carbonées étant non substituées ou substituées, chacune indépendamment, par 1, 2, 3, 4, 5, 6, 7, 8 ou 9 atomes de fluor,
R3 et R7 ne représentant pas l'un et l'autre simultanément des atomes d'hydrogène ;
R8 représente H, un groupe alkyle ayant 1, 2, 3 ou 4 atomes de carbone ou cycloalkyle ayant 3, 4 ou 5 atomes de carbone, les chaînes carbonées ou les radicaux cycloalkyle étant non substitués ou substitués par 1, 2, 3, 4, 5, 6, 7, 8 ou 9 atomes de fluor ;
ainsi que leurs sels pharmaceutiquement acceptables et leurs sels avec l'acide trifluoroacétique.

2. Composés de formule I selon la revendication 1, dans lesquels :
R1 et R2 forment ensemble, avec les deux atomes de carbone auxquels ils sont fixés, un cycle carboné à cinq, six, sept ou huit chaînons, comportant une ou deux doubles liaisons,
le cycle étant non substitué ou substitué par 1, 2, 3, 4, 5, 6, 7, 8, 9, 10, 11 ou 12 atomes de fluor et/ou par un ou deux radicaux choisis dans l'ensemble constitué par CH₃ et OCH₃ ;
R3, R4, R5, R6 et R7 représentent, indépendamment les uns des autres, H, F, Cl, Br, un groupe alkyle ayant 1, 2, 3 ou 4 atomes de carbone ou alcoxy ayant 1, 2, 3 ou 4 atomes de carbone,
les chaînes carbonées étant non substituées ou substituées, chacune indépendamment, par 1, 2, 3, 4, 5, 6, 7, 8 ou 9 atomes de fluor,
R3 et R7 ne représentant pas l'un et l'autre simultanément des atomes d'hydrogène ;
R8 représente H, un groupe alkyle ayant 1, 2, 3 ou 4 atomes de carbone ou cycloalkyle ayant 3, 4 ou 5 atomes de carbone, les chaînes carbonées ou les radicaux cycloalkyle étant non substitués ou substitués par 1, 2, 3, 4, 5, 6, 7, 8 ou 9 atomes de fluor ;
ainsi que leurs sels pharmaceutiquement acceptables et leurs sels avec l'acide trifluoroacétique.

3. Composés de formule I selon la revendication 2, dans lesquels :
R1 et R2 forment ensemble, avec les deux atomes de carbone auxquels ils sont fixés, un cycle carboné à cinq, six ou sept chaînons, comportant une double liaison,
R3 et R7 représentent, indépendamment l'un de l'autre, F, Cl, Br ou un groupe alkyle ayant 1, 2, 3 ou 4 atomes de carbone,
les chaînes carbonées étant non substituées ou substituées, chacune indépendamment, par 1, 2, 3, 4, 5, 6, 7, 8 ou 9 atomes de fluor,
R4, R5 et R6 représentent H,
R8 représente H ou un groupe alkyle ayant 1, 2, 3 ou 4 atomes de carbone, les chaînes carbonées étant non substitués ou substitués par 1, 2, 3, 4, 5, 6, 7, 8 ou 9 atomes de fluor ;
ainsi que leurs sels pharmaceutiquement acceptables et leurs sels avec l'acide trifluoroacétique.

4. Composés selon la revendication 3, **caractérisés en ce qu'**ils sont choisis dans le groupe constitué par :
la (2,6-dichlorophényl)-(4,5,6,7-tétrahydro-1 H-benzo-imidazol-2-yl)amine et
la (2,6-dichlorophényl)-méthyl-(4,5,6,7-tétrahydro-1H-benzo-imidazol-2-yl)amine ainsi que leurs sels pharmaceutiquement acceptables et leurs sels avec l'acide trifluoroacétique.

5. Composés de formule I et leurs sels pharmaceutiquement acceptables selon une ou plusieurs des revendications 1 à 4, pour utilisation en tant que médicament.

6. Utilisation d'un composé de formule I et/ou de ses sels pharmaceutiquement acceptables selon une ou plusieurs des revendications 1 à 4, pour la fabrication d'un médicament destiné au traitement ou à la prophylaxie de troubles de l'impulsion respiratoire, de troubles respiratoires, de troubles respiratoires dus au sommeil, d'apnées du sommeil, du ronflement, de néphropathies aiguës et chroniques, de l'insuffisance rénale aiguë et de l'insuffisance rénale chronique, de troubles de la fonction intestinale, de l'hypertension artérielle, de l'hypertonie essentielle, de maladies du système nerveux central, de maladies qui résultent d'une hyperexcitabilité du SNC, de l'épilepsie et de convulsions d'origine centrale, ou d'états anxieux, de dépressions et de psychoses, d'états ischémiques du système nerveux périphérique ou central ou de l'accident vasculaire cérébral, de lésions et de maladies aiguës et chroniques d'organes périphériques ou des membres qui sont provoquées par des incidents d'ischémie ou de reperfusion, de l'athérosclérose, de troubles du métabolisme des lipides, de thromboses, de troubles de la fonction biliaire, de l'attaque par des ectoparasites, de maladies faisant suite à un dysfonctionnement endothélial, de maladies qui sont provoquées par des protozoaires, du paludisme, pour la conservation et le stockage de transplants pour des gestes chirurgicaux, pour l'utilisation dans des interventions chirurgicales et des greffes d'organes ou pour le traitement d'états de choc ou du diabète et de lésions tardives diabétiques ou de maladies dans lesquelles la prolifération cellulaire représente une cause primaire ou secondaire et pour le maintien de la santé et le prolongement de la vie.

7. Utilisation d'un composé de formule 1 et/ou de ses sels pharmaceutiquement acceptables selon une ou plusieurs des revendications 1 à 4, en association avec d'autres principes actifs ou substances actives pour la fabrication d'un médicament destiné au traitement ou à la prophylaxie de troubles de l'impulsion respiratoire, de troubles respiratoires, de troubles respiratoires dus au sommeil, d'apnées du sommeil, du ronflement, de néphropathies aiguës et chroniques, de l'insuffisance rénale aiguë et de l'insuffisance rénale chronique, de troubles de la fonction intestinale, de l'hypertension artérielle, de l'hypertonie essentielle, de maladies du système nerveux central, de maladies qui résultent d'une hyperexcitabilité du SNC, de l'épilepsie et de convulsions d'origine centrale, ou d'états anxieux, de dépressions et de psychoses, d'états ischémiques du système nerveux périphérique ou central ou de l'accident vasculaire cérébral, de lésions et de maladies aiguës et chroniques d'organes périphériques ou des membres qui sont provoquées par des incidents d'ischémie ou de reperfusion, de l'athérosclérose, de troubles du métabolisme des lipides, de thromboses, de troubles de la fonction biliaire, de l'attaque par des ectoparasites, de maladies faisant suite à un dysfonctionnement endothélial, de maladies qui sont provoquées par des protozoaires, du paludisme, pour la conservation et le stockage de transplants pour des gestes chirurgicaux, pour l'utilisation dans des interventions chirurgicales et des greffes d'organes ou pour le traitement d'états de choc ou du diabète et de lésions tardives diabétiques ou de maladies dans lesquelles la prolifération cellulaire représente une cause primaire ou secondaire et pour le maintien de la santé et le prolongement de la vie.

8. Utilisation d'un composé de formule I et/ou de ses sels pharmaceutiquement acceptables selon une ou plusieurs des revendications 1 à 4, seul ou en association avec d'autres principes actifs ou substances actives, pour la fabrication d'un médicament destiné au traitement ou à la prophylaxie de troubles de l'impulsion respiratoire et/ou de troubles respiratoires dus au sommeil et d'apnées du sommeil.

9. Utilisation d'un composé de formule I et/ou de ses sels pharmaceutiquement acceptables selon une ou plusieurs des revendications 1 à 4, seul ou en association avec d'autres principes actifs ou substances actives, pour la fabrication d'un médicament destiné au traitement ou à la prophylaxie du ronflement.

10. Utilisation d'un composé de formule I et/ou de ses sels pharmaceutiquement acceptables selon une ou plusieurs des revendications 1 à 4, seul ou en association avec d'autres principes actifs ou substances actives, pour la fabrication d'un médicament destiné au traitement ou à la prophylaxie de néphropathies aiguës et chroniques, de l'insuffisance rénale aiguë ou de l'insuffisance rénale chronique.

11. Utilisation d'un composé de formule I et/ou de ses sels pharmaceutiquement acceptables selon une ou plusieurs des revendications 1 à 4, seul ou en association avec d'autres principes actifs ou substances actives, pour la fabrication d'un médicament destiné au traitement ou à la prophylaxie de troubles de la fonction intestinale.

12. Médicament pour l'utilisation humaine, vétérinaire ou phytoprotectrice, contenant une quantité efficace d'un ou plusieurs composés de formule I et/ou de ses(leurs) sels pharmaceutiquement acceptables selon une ou plusieurs des revendications 1 à 4.

13. Médicament pour l'utilisation humaine, vétérinaire ou phytoprotectrice, contenant une quantité efficace d'un ou plusieurs composés de formule I et/ou de ses(leurs) sels pharmaceutiquement acceptables selon une ou plusieurs des revendications 1 à 4, en association avec un ou plusieurs autres principes actifs pharmacologiques ou une ou plusieurs substances actives pharmacologiques.

14. Utilisation de composés de formule I 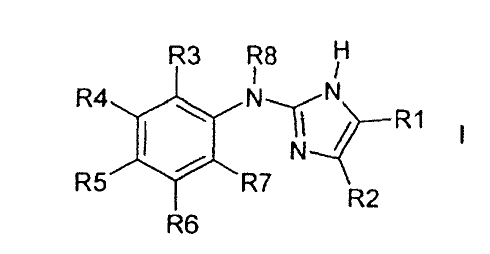 et/ou de leurs sels pharmaceutiquement acceptables, pour la fabrication d'un médicament destiné au traitement de maladies qui peuvent être influencées par des inhibiteurs de NHE (échangeur sodium/proton), formule dans laquelle :
R1 et R2 forment ensemble, avec les deux atomes de carbone auxquels ils sont fixés, un cycle carboné à cinq, six, sept ou huit chaînons, comportant une ou deux doubles liaisons,
le cycle étant non substitué ou substitué par 1, 2, 3, 4, 5, 6, 7, 8, 9, 10, 11 ou 12 atomes de fluor et/ou par un ou deux radicaux choisis dans l'ensemble constitué par NR9R10, alkyle ayant 1, 2, 3 ou 4 atomes de carbone, OH, CN, CF₃ ou alcoxy ayant 1, 2, 3 ou 4 atomes de carbone ;
R9 et R10 représentent, indépendamment l'un de l'autre, H ou un groupe alkyle ayant 1, 2, 3 ou 4 atomes de carbone ;
R3, R4, R5, R6 et R7 représentent, indépendamment les uns des autres, H, F, Cl, Br, l, un groupe alkyle ayant 1, 2, 3 ou 4 atomes de carbone, alcényle en C₂-C₄, cycloalkyle ayant 3, 4, 5 ou 6 atomes de carbone, OH, alcoxy ayant 1, 2, 3 ou 4 atomes de carbone, CN, NO₂, NH₂, alkylamino ayant 1, 2, 3 ou 4 atomes de carbone ou dialkylamino dont chaque fragment alkyle a 1, 2, 3 ou 4 atomes de carbone,
les chaînes carbonées ou les cycles carbonés étant non substitués ou substitués, chacun indépendamment, par 1, 2, 3, 4, 5, 6, 7, 8 ou 9 atomes de fluor, R3 et R7 ne représentant pas l'un et l'autre simultanément des atomes d'hydrogène ;
R8 représente H, un groupe alkyle ayant 1, 2, 3 ou 4 atomes de carbone ou cycloalkyle ayant 3, 4 ou 5 atomes de carbone, les chaînes carbonées ou les cycles carbonés étant non substitués ou substitués par 1, 2, 3, 4, 5, 6, 7, 8 ou 9 atomes de fluor.

15. Utilisation d'un composé de formule I et/ou de ses sels pharmaceutiquement acceptables selon la revendication 14, pour la fabrication d'un médicament destiné au traitement ou à la prophylaxie de troubles de l'impulsion respiratoire, de troubles respiratoires et de troubles respiratoires dus au sommeil, tels que les apnées du sommeil, du ronflement, de néphropathies aiguës et chroniques, de l'insuffisance rénale aiguë et de l'insuffisance rénale chronique, de troubles de la fonction intestinale, de maladies du système nerveux central, de maladies qui résultent d'une hyperexcitabilité du SNC, de l'épilepsie et de convulsions d'origine centrale, d'états anxieux, de dépressions et de psychoses, d'états ischémiques du système nerveux périphérique et central, et de l'accident vasculaire cérébral, de lésions et de maladies aiguës et chroniques d'organes périphériques et des membres qui sont provoquées par des incidents d'ischémie ou de reperfusion, pour la conservation et le stockage de transplants pour des gestes chirurgicaux, pour l'utilisation dans des interventions chirurgicales et des greffes d'organes, pour le traitement d'états de choc, pour le traitement du diabète et de lésions tardives diabétiques, de l'athérosclérose, de troubles du métabolisme des lipides, de maladies dans lesquelles la prolifération cellulaire représente une cause primaire ou secondaire, de thromboses, de troubles de la fonction biliaire, pour le traitement ou la prophylaxie de l'attaque par des ectoparasites, pour le traitement ou la prophylaxie de maladies faisant suite à un dysfonctionnement endothélial, pour le maintien de la santé et le prolongement de la vie et pour le traitement ou la prophylaxie de maladies causées par des protozoaires et du paludisme.

16. Procédé pour la préparation d'α-aminocétals de formule V 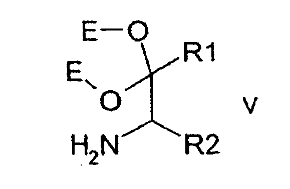 dans laquelle
R1 et R2 forment ensemble, avec les deux atomes de carbone auxquels ils sont fixés, un cycle carboné à cinq à huit chaînons, saturé ou comportant une double liaison,
le cycle étant non substitué ou substitué par 1, 2, 3, 4, 5, 6, 7, 8, 9, 10, 11 ou 12 atomes de fluor et/ou par un ou deux radicaux choisis dans l'ensemble constitué par NR11R12, alkyle ayant 1, 2, 3 ou 4 atomes de carbone, CN, CF₃ ou alcoxy ayant 1, 2, 3 ou 4 atomes de carbone ;
R11, R12 représentent, indépendamment l'un de l'autre, un groupe alkyle ayant 1, 2, 3 ou 4 atomes de carbone, benzyle, 4-méthoxybenzyle ;
E représente un groupe alkyle ayant 1, 2, 3 ou 4 atomes de carbone ou les deux radicaux E forment un cétal cyclique dans lequel E-E représente un groupe alkylène en C₂-C₄ ;
Hal est Cl, Br, l
**caractérisé en ce qu'**on convertit une α-halogénocétone ou un α-halogénoaldéhyde de formule VI, à l'aide d'un azide, par échange azide-halogène, en l'α-azide-cétone correspondante ou l'α-azide-aldéhyde correspondant de formule VII 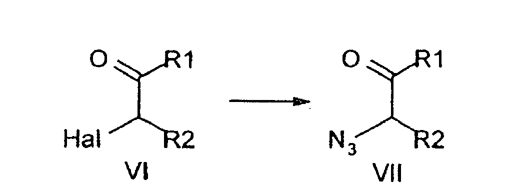 que l'on convertit ensuite à l'aide d'un alcool mono- ou dihydrique, avec cétalisation ou acétalisation, en l'α-azide-cétal ou -acétal de formule VIII, 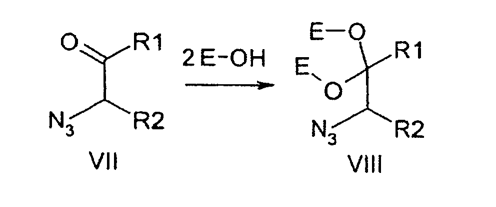 qui est ensuite réduit en composés de formule V.
